(19) Europäisches Patentamt
European Patent Office
Office européen des brevets

(11) **EP 4 717 782 A2**

(12) **EUROPEAN PATENT APPLICATION**

(43) Date of publication:
01.04.2026 Bulletin 2026/14

(21) Application number: 25221455.6

(22) Date of filing: 31.08.2022

(51) International Patent Classification (IPC):
*C12Q 1/6841* (2018.01)

(52) Cooperative Patent Classification (CPC):
(C-Sets available)
C12Q 1/6841                                              (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 01.09.2021 US 202163239897 P

(62) Document number(s) of the earlier application(s) in
accordance with Art. 76 EPC:
**22777544.2 / 4 396 373**

(71) Applicant: **Becton, Dickinson and Company**
**Franklin Lakes, NJ 07417-1880 (US)**

(72) Inventors:
• **SONG, Hye-Won**
**San Jose, CA 95131 (US)**

• **MARTIN, Jody**
**San Jose, CA 95131 (US)**
• **NAKAMOTO, Margaret**
**San Jose, CA 95131 (US)**

(74) Representative: **Carpmaels & Ransford LLP**
**One Southampton Row**
**London WC1B 5HA (GB)**

Remarks:
•This application was filed on 08.12.2025 as a
divisional application to the application mentioned
under INID code 62.
•Claims filed after the date of filing of the application
/after the date of receipt of the divisional
application(Rule 68(4) EPC).

(54) **SPATIAL MULTIOMICS USING IN SITU REVERSE TRANSCRIPTION**

(57) Disclosed herein include systems, methods, compositions, and kits for determining the spatial location and copy number of targets (e.g., nucleic acid targets, cellular component targets) in a sample. There are provided, in some embodiments, substrates comprising a plurality of spatial regions. A plurality of oligonucleotide barcodes can be associated with each of the spatial regions and can comprise a predetermined spatial label. Oligonucleotide barcodes of the same spatial region can comprise the same spatial label, and oligonucleotide barcodes of the different spatial regions can comprise different spatial labels. The method can comprise contacting the substrate with a sample such that each distinct spatial region contacts a distinct spatial location of the sample. The method can comprise in situ extension (e.g., reverse transcription) of the oligonucleotide barcodes.

| bead | 5' amine | Universal label | Dimension label | Sample label | Cell label | Spatial label | Molecular label | Target-binding region |
|------|----------|-----------------|-----------------|--------------|------------|---------------|-----------------|-----------------------|

108

104

*FIG. 1*

EP 4 717 782 A2

(52) Cooperative Patent Classification (CPC): (Cont.)

C-Sets
**C12Q 1/6841, C12Q 2525/155, C12Q 2525/173,
C12Q 2525/179, C12Q 2535/122, C12Q 2537/16,
C12Q 2537/163, C12Q 2543/10, C12Q 2545/10,
C12Q 2563/179, C12Q 2565/514**

**Description**

RELATED APPLICATIONS

This application claims the benefit under 35 U.S.C. §119(e) of U.S. Provisional Patent Application Ser. No. 63/239,897, filed September 1, 2021, the content of this related application is incorporated herein by reference in its entirety for all purposes.

BACKGROUND

Field

The present disclosure relates generally to the field of molecular biology, for example determining gene expression using molecular barcoding.

Description of the Related Art

Methods and techniques such as in situ hybridization and immunohistochemistry allow the visualization of the locations of target molecules within the sample. Methods and techniques for labeling target molecules for amplification and sequencing, for example stochastic barcoding, are useful for determining the identities of the target molecules. Determining the identities and locations of the targets molecules in the sample is important for clinical applications, diagnostics, and biomedical research. Thus, there is a need for methods and techniques capable of correlating the identities of the target molecules with the locations of target molecules within the sample.

SUMMARY

Disclosed herein include methods for determining the spatial location and copy number of a nucleic acid target in a sample. In some embodiments, the method comprises: providing a substrate comprising a plurality of spatial regions, wherein a plurality of oligonucleotide barcodes are associated with each of the spatial regions, wherein each of the oligonucleotide barcodes comprises a universal sequence, a molecular label, a target-binding region capable of hybridizing to a nucleic acid target, and a predetermined spatial label, wherein oligonucleotide barcodes of the same spatial region comprise the same spatial label, and wherein oligonucleotide barcodes of the different spatial regions comprise different spatial labels. The method can comprise: contacting the substrate with a sample comprising copies of a nucleic acid target, wherein the contacting comprises contacting the plurality of the spatial regions with the sample such that each distinct spatial region contacts a distinct spatial location of the sample. The method can comprise: extending the plurality of oligonucleotide barcodes hybridized to the copies of a nucleic acid target to generate a plurality of barcoded nucleic acid molecules each comprising a sequence complementary to at least a portion of the nucleic acid target. The method can comprise: obtaining sequencing data comprising a plurality of sequencing reads of the plurality of barcoded nucleic acid molecules, or products thereof, wherein each of the plurality of sequencing reads comprises a spatial label sequence, a molecular label sequence, and a subsequence of the nucleic acid target. The method can comprise: for each unique spatial label sequence, which is associated a distinct spatial region of the substrate and thereby a distinct spatial location of the sample, counting the number of molecular labels with distinct sequences associated with a nucleic acid target to determine the copy number of the nucleic acid target at each spatial location of the sample.

Disclosed herein include methods for determining the spatial location and copy number of cellular component targets in a sample. The method can comprise: contacting a plurality of cellular component-binding reagents with the sample, wherein each of the plurality of cellular component-binding reagents comprises a cellular component-binding reagent specific oligonucleotide comprising a unique identifier sequence for the cellular component-binding reagent, and wherein the cellular component-binding reagent is capable of specifically binding to at least one of the plurality of cellular component targets. The method can comprise: extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence. The method can comprise: obtaining sequencing data comprising a plurality of sequencing reads of the plurality of barcoded cellular component-binding reagent specific oligonucleotides, or products thereof, wherein each of the plurality of sequencing reads comprises a spatial label sequence, a molecular label sequence, and at least a portion of the unique identifier sequence. The method can comprise: for each unique spatial label sequence, which is associated a distinct spatial region of the substrate and thereby a distinct spatial location of the sample, counting the number of molecular labels with distinct sequences associated with a unique identifier sequence to determine the number of copies of at least one cellular component target of the plurality of cellular component targets at each spatial location of the sample.

In some embodiments, at least a portion of the contacting step is performed in the presence in the presence of extension reagents, optionally the entire contacting step is performed in the presence in the presence of the extension reagents, further optionally the contacting and extension steps are simultaneous, optionally the sample and substrate are in contact during the extension step, optionally the extension is performed in situ. In some embodiments, the extension reagents comprise reverse transcription reagents, optionally reverse transcription reagents comprise a reverse transcriptase and dNTPs. In some embodiments, the method comprises a denaturing step, optionally denaturing separates the nucleic acid target from the barcoded nucleic acid molecule and/or the cellular component-binding reagent specific oligonucleotide from the barcoded cellular component-binding reagent specific oligonucleotide. In some embodiments, the contacting step comprises an overlay of the substrate on the sample. The method can comprise: separating the substrate from the sample from the substrate after the contacting step.

In some embodiments, the method (i) comprises fixing the sample prior to the contacting step or (ii) does not comprise fixing the sample prior to the contacting step. In some embodiments, the sample is physically divided or is intact during the contacting step. In some embodiments, the spatial locations of the nucleic acid targets in the sample are on a surface of the sample, inside the sample, subcellularly in the sample, or any combination thereof. In some embodiments, the sample comprises a plurality of cells, optionally the nucleic acid targets are associated with the plurality of cells. In some embodiments, the plurality of cells comprise one or more cell types, optionally said one or more cell types are selected from the group consisting of: brain cells, heart cells, cancer cells, circulating tumor cells, organ cells, epithelial cells, metastatic cells, benign cells, primary cells, and circulatory cells, or any combination thereof. In some embodiments, the sample comprises a tissue, a cell monolayer, fixed cells, a tissue section, or any combination thereof. In some embodiments, the sample comprises a biological sample, a clinical sample, an environmental sample, a biological fluid, a tissue, a tissue section derived from a subject, or any combination thereof, optionally the subject is a human, a mouse, a dog, a rat, or a vertebrate.

The method can comprise: determining genotype, phenotype, or one or more genetic mutations of the subject based on the spatial location of the nucleic acid targets in the sample. The method can comprise: predicting susceptibility of the subject to one or more diseases. In some embodiments, at least one of the one or more diseases is cancer or a hereditary disease. The method can comprise: determining cell types of the plurality of cells in the sample. In some embodiments, a drug is chosen based on predicted responsiveness of the cell types of the plurality of cells in the sample.

The method can comprise: imaging the sample, optionally imaging the sample after the contacting step, optionally the imaging generates imaging data. In some embodiments, imaging the sample comprises staining the sample with a stain, wherein the stain is a fluorescent stain, a negative stain, an antibody stain, or any combination thereof, optionally staining comprises Immunocytochemistry (ICC), Immunohistochemistry (IHC), Immunofluorescence (IF), or any combination thereof. In some embodiments, imaging comprises microscopy, confocal microscopy, time-lapse imaging microscopy, fluorescence microscopy, multi-photon microscopy, quantitative phase microscopy, surface enhanced Raman spectroscopy, videography, manual visual analysis, automated visual analysis, or any combination thereof. The method can comprise: associating the imaging data and sequencing data of one or more spatial locations of the sample. The method can comprise: correlation analysis of the imaging data and the sequencing data of the spatial locations, optionally the correlation analysis identifies one or more of the following: candidate biomarkers, candidate therapeutic agents, candidate doses of therapeutic agents, and/or cellular targets of candidate therapeutic agents. In some embodiments, said imaging produces an image that is used to construct a map of a physical representation of said sample, optionally said map is two dimensional or three dimensional. The method can comprise: mapping the nucleic acid targets and/or cellular component targets onto the map of the sample. The method can comprise: isolating one or more barcoded nucleic acid molecules corresponding to a spatial location of interest, optionally the isolating comprises selective hybridization and pull-down of one or more barcoded nucleic acid molecules comprising the unique spatial label of a spatial region corresponding to a spatial location of interest.

In some embodiments, the oligonucleotide barcode comprises a target-binding region comprising a capture sequence, optionally the target-binding region comprises a poly(dT) region. In some embodiments, the cellular component-binding reagent specific oligonucleotide comprises a sequence complementary to the capture sequence configured to capture the cellular component-binding reagent specific oligonucleotide, optionally the sequence complementary to the capture sequence comprises a poly(dA) region. In some embodiments, the nucleic acid target comprises a nucleic acid molecule. In some embodiments, the nucleic acid molecule comprises ribonucleic acid (RNA), messenger RNA (mRNA), microRNA, small interfering RNA (siRNA), RNA degradation product, RNA comprising a poly(A) tail, a sample indexing oligonucleotide, a cellular component-binding reagent specific oligonucleotide, or any combination thereof. In some embodiments, each of the spatial regions comprises a plurality of oligonucleotide barcodes configured to hybridize to a panel of nucleic acid targets. In some embodiments, the cellular component-binding reagent specific oligonucleotide comprises a second universal sequence. In some embodiments, obtaining sequence information of the plurality of barcoded cellular component-binding reagent specific oligonucleotides, or products thereof, comprises: amplifying the plurality of barcoded cellular component-binding reagent specific oligonucleotides, or products thereof, using a primer capable of hybridizing to the first universal sequence, or a complement thereof, and a primer capable of hybridizing to the second universal sequence, or a complement thereof, to generate a plurality of amplified barcoded cellular component-binding reagent specific oligonucleotides; and obtaining sequencing data of the plurality of amplified barcoded cellular component-binding reagent specific oligonucleotides, or products thereof. In some embodiments, obtaining sequencing data comprises attaching sequencing adaptors to (i) the plurality of barcoded nucleic acid molecules, or products thereor, and/or (ii) the plurality of barcoded cellular component-binding reagent specific oligonucleotides, or products thereof.

The method can comprise: after contacting the plurality of cellular component-binding reagents with the sample, removing one or more cellular component-binding reagents of the plurality of cellular component-binding reagents that are not contacted with the sample, optionally removing the one or more cellular component-binding reagents not contacted with the sample comprises: removing the one or more cellular component-binding reagents not contacted with the respective at least one of the plurality of cellular component targets. In some embodiments, the cellular component target comprises an intracellular protein, a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof.

In some embodiments, extending the plurality of oligonucleotide barcodes comprises extending the plurality of oligonucleotide barcodes using a reverse transcriptase and/or a DNA polymerase lacking at least one of 5' to 3' exonuclease activity and 3' to 5' exonuclease activity, optionally the DNA polymerase comprises a Klenow Fragment, further optionally the reverse transcriptase comprises a viral reverse transcriptase, optionally wherein the viral reverse transcriptase is a murine leukemia virus (MLV) reverse transcriptase or a Moloney murine leukemia virus (MMLV) reverse transcriptase. In some embodiments, less than about 5 percent of nucleic acid targets are mapped to an incorrect spatial location due to diffusion of said nucleic acid target from a starting spatial location.

In some embodiments, wherein each of the plurality of oligonucleotide barcodes comprises a substrate linker functional group, each of the plurality of substrates comprises a substrate functional group, and the substrate functional group and the substrate linker functional group are associated with each other. In some embodiments, the substrate linker functional group and the substrate functional group are individually selected from the group consisting of C6, biotin, streptavidin, primary amine(s), aldehyde(s), ketone(s), and any combination thereof. In some embodiments, the oligonucleotide barcode is associated with the substrate through a substrate linker. In some embodiments, the substrate linker comprises a carbon chain, optionally the carbon chain comprises 2-30 carbons, and further optionally the carbon chain comprises 12 carbons. In some embodiments, the substrate linker comprises 5' amino modifier C12 (5AmMC12), or a derivative thereof. In some embodiments, the spatial label is 6-60 nucleotides in length. In some embodiments, the oligonucleotide barcode is 50-500 nucleotides in length. In some embodiments, the oligonucleotide barcode is attached to the substrate. In some embodiments, the oligonucleotide barcode is covalently attached to the substrate. In some embodiments, the oligonucleotide barcode is conjugated to the substrate. In some embodiments, the oligonucleotide barcode is conjugated to the substrate through a chemical group selected from the group consisting of a UV photocleavable group, a streptavidin, a biotin, an amine, and a combination thereof. In some embodiments, the oligonucleotide barcode is non-covalently attached to the substrate.

In some embodiments, the oligonucleotide barcode is configured to be detachable from the substrate, The method can comprise: dissociating the oligonucleotide barcode from the substrate, and optionally dissociating the oligonucleotide barcode from the substrate comprises detaching the oligonucleotide barcode from the substrate by UV photocleaving, chemical treatment, heating, enzyme treatment, or any combination thereof. In some embodiments, the dissociating occurs after barcoding the oligonucleotide barcodes.

In some embodiments, the oligonucleotide barcode is configured to be non-detachable from the substrate. In some embodiments, the oligonucleotide barcode is conjugated to the substrate by a 1,3-dipolar cycloaddition reaction, a hetero-Diels-Alder reaction, a nucleophilic substitution reaction, a non-aldol type carbonyl reaction, an addition to carbon-carbon multiple bond, an oxidation reaction, a click reaction, or any combination thereof. In some embodiments, at least one oligonucleotide barcode of the plurality of oligonucleotide barcodes is immobilized on the substrate, partially immobilized on the substrate, enclosed in the substrate, partially enclosed in the substrate, or a combination thereof. In some embodiments, the substrate comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof. In some embodiments, the target-binding region comprises a gene-specific sequence, an oligo(dT) sequence, a random multimer, or any combination thereof. In some embodiments, each spatial label of the plurality of oligonucleotide barcodes comprise at least 6 nucleotides. In some embodiments, each molecular label of the plurality of oligonucleotide barcodes comprises at least 6 nucleotides.

In some embodiments, the substrate comprises a solid support. In some embodiments, the solid support comprises a planar surface. In some embodiments, the substrate comprises an oligonucleotide array comprising a plurality of spots, and wherein each spot of the oligonucleotide array comprises a distinct spatial region of the plurality of spatial regions. In some embodiments, the substrate comprises a microwell array, and wherein each microwell of the microwell array comprises a distinct spatial region of the plurality of spatial regions.

The method can comprise: prior to contacting a plurality of cellular component-binding reagents with the sample and/or contacting the substrate with the sample, contacting the sample with a blocking reagent, one or more decoy oligonucleotides, and/or one or more blocking oligonucleotides. In some embodiments, contacting a plurality of cellular component-binding reagents with the sample is conducted in the presence of a blocking reagent. In some embodiments, the blocking reagent comprises a plurality of oligonucleotides complementary to at least a portion of the cellular component-binding reagent specific oligonucleotides. In some embodiments, the blocking reagent comprises an antibody or a fragment thereof derived from a first species, and wherein the blocking reagent comprises sera derived from the first species. In some embodiments, the sample comprises one or more non-target nucleic acids, wherein the blocking reagent comprises a plurality of decoy oligonucleotides capable of hybridizing to at least one of the one or more non-target nucleic acids. In some embodiments, each of the plurality of decoy oligonucleotides are capable of hybridizing to at least a portion of a non-target nucleic acid. In some embodiments, the decoy oligonucleotide comprises a sequence complementary to at least a portion of a non-target nucleic acid. In some embodiments, the decoy oligonucleotide comprises a sequence identical to or substantially similar to a sequence of the cellular component-binding reagents specific oligonucleotides. In some embodiments, the sequence is 3-40 nucleotides in length. In some embodiments, the decoy oligonucleotide has at most 50% sequence identity to the cellular component-binding reagent specific oligonucleotides. In some embodiments, the decoy oligonucleotide does not comprise a UMI. In some embodiments, the decoy oligonucleotide comprises a random sequence, and optionally the random sequence is about four, five, six, seven, eight, nine, ten, eleven, twelve, thirteen, fourteen, or fifteen nucleotides in length. In some embodiments, the decoy oligonucleotide does not comprise any sequence having more than four, five, six, or seven consecutive Ts or As. In some embodiments, the decoy oligonucleotide comprise at least one G or C in every four, five, six, or seven consecutive nucleotides. In some embodiments, the decoy oligonucleotide comprises one or more modified nucleotides. In some embodiments, the decoy oligonucleotide comprises a 5' modification, and optionally the 5' modification comprises a 5' Amino Modifier C12 modification (5AmMC12). In some embodiments, the decoy oligonucleotide comprises a 3' modification, and optionally the 3' modification comprises a 3' dideoxy-C modification (ddC). In some embodiments, the decoy oligonucleotide is 30 to 65 nucleotides in length.

In some embodiments, the sample comprises one or more undesirable nucleic acid species, the method comprising: contacting a blocking oligonucleotide with the sample, wherein the blocking oligonucleotide specifically binds to at least one of the one or more undesirable nucleic acid species; whereby the extension of the at least one of the one or more undesirable nucleic acid species is reduced by the blocking oligonucleotide In some embodiments, the blocking oligonucleotide is contacted with the sample before the substrate is contacted with the sample. In some embodiments, the blocking oligonucleotide is contacted with the sample after the substrate is contacted with the sample. In some embodiments, the blocking oligonucleotide is contacted with the sample when the substrate is contacted with the sample. In some embodiments, the blocking oligonucleotide comprises: (i) a sequence that specifically binds to the at least one of the one or more undesirable nucleic acid species, and (ii) the sequence, or a subsequence, of the target binding region. In some embodiments, the blocking oligonucleotide comprises (i) a sequence that specifically binds to the at least one of the one or more undesirable nucleic acid species, (ii) the sequence, or a subsequence, of the target binding region, and (iii) a sequence that does not hybridize to the at least one of the one or more undesirable nucleic acid species. In some embodiments, the blocking oligonucleotide comprises a 3' non-annealing region configured to not anneal to the one or more undesirable nucleic acid species; and optionally the non-complementarity between the 3' non-annealing region and the region of the undesirable nucleic acid species 5' adjacent to the sequence specifically bound by the blocking oligonucleotide is at least 50%, is at least 60%, is at least 70%, is at least 80%, is at least 90%, is at least 95%, or is about 100%. In some embodiments, the 3' non-annealing region is 1 nt to 100 nt long, is 1 nt to 50 nt long, is 1 nt to 21 nt long, is 1 nt to 10 nt long, or is about 5 nt long. In some embodiments, the blocking oligonucleotide is a locked nucleic acid (LNA), a peptide nucleic acid (PNA), a DNA, an LNA/PNA chimera, an LNA/DNA chimera, or a PNA/DNA chimera. In some embodiments, the blocking oligonucleotide does not comprise non-natural nucleotides.

The method can comprise: providing blocking oligonucleotides that specifically bind to two or more undesirable nucleic acid species, optionally at least 10 or to at least 100 undesirable nucleic acid species, in the sample. In some embodiments, the blocking oligonucleotide has a $T_m$ of at least 50 °C, of at least 60 °C, or of at least 70 °C. In some embodiments, the blocking oligonucleotide is unable to function as a primer for a reverse transcriptase or a polymerase. In some embodiments, the blocking oligonucleotide is 8 nt to 100 nt long, is 10 nt to 50 nt long, is 12 nt to 21 nt long, is 20 nt to 30 nt long, or is about 25 nt long. In some embodiments, the one or more undesirable nucleic acid species amounts to about 50%, to about 60%, to about 70%, or to about 80% of the nucleic acid content of the sample. In some embodiments, the undesirable nucleic acid species is selected from the group consisting of ribosomal RNA, mitochondrial RNA, genomic DNA, intronic sequence, high abundance sequence, and a combination thereof. In some embodiments, the blocking oligonucleotides specifically binds to within 100 nt, to within 50 nt, or to within 25 nt of the 3' end of the one or more undesirable nucleic acid species. In some embodiments, the blocking oligonucleotide specifically binds to within 100 nt of the 5' end of the one or more undesirable nucleic acid species, or the blocking oligonucleotide specifically binds to within 100 nt of the middle of the one or more undesirable nucleic acid species. In some embodiments, the one or more undesirable nucleic acid species are mRNA molecules and the blocking oligonucleotide specific binds to within 10 nt of the 3' poly(A) tail of the one or more undesirable nucleic acid species.

Disclosed herein include compositions. In some embodiments, the composition comprises: a micro-well array comprising a plurality of spatial regions, wherein the micro-well array comprises at least 100 micro-wells, wherein each micro-well has a volume ranging from about 10 $\mu m^3$ to about 786,000 $\mu m^3$, wherein each micro-well comprises a distinct spatial region, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

Disclosed herein include compositions. In some embodiments, the composition comprises: an oligonucleotide array comprising a plurality of spatial regions, wherein the oligonucleotide array comprises at least 100 spots, wherein each spot of the oligonucleotide array comprises a distinct spatial region of the plurality of spatial regions, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

The composition can comprise: a cartridge, wherein the cartridge comprises at least one of: an inlet port, an outlet port, a pump, a valve, a vent, a reservoir, a sample collection chamber, a temperature control apparatus, or any combination thereof. The composition can comprise: a buffer. The composition can comprise: one or more reagents for a reverse transcription reaction, one or more reagents for an amplification reaction, or both. In some embodiments, the cartridge comprises a transparent window for optical imaging of the at least 100 micro-wells.

BRIEF DESCRIPTION OF THE DRAWINGS

FIG. 1 illustrates a non-limiting exemplary barcode.
FIG. 2 shows a non-limiting exemplary workflow of barcoding and digital counting.
FIG. 3 is a schematic illustration showing a non-limiting exemplary process for generating an indexed library of targets barcoded at the 3'-ends from a plurality of targets.
FIGS. 4A-4C depict a non-limiting exemplary spatial multiomics workflow.
FIG. 5. depicts a non-limiting exemplary spatial multiomics platform.

DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

All patents, published patent applications, other publications, and sequences from GenBank, and other databases referred to herein are incorporated by reference in their entirety with respect to the related technology.

Quantifying small numbers of nucleic acids, for example messenger ribonucleotide acid (mRNA) molecules, is clinically important for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs)) can be used to count the number of molecules and correct for amplification bias. Stochastic barcoding, such as the Precise™ assay (Cellular Research, Inc. (Palo Alto, CA)) and Rhapsody™ assay (Becton, Dickinson and Company (Franklin Lakes, NJ)), can correct for bias induced by PCR and library preparation steps by using molecular labels (MLs) to label mRNAs during reverse transcription (RT).

The Precise™ assay can utilize a non-depleting pool of stochastic barcodes with large number, for example 6561 to 65536, unique molecular label sequences on poly(T) oligonucleotides to hybridize to all poly(A)-mRNAs in a sample during the RT step. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate can be pooled into a single tube for PCR amplification and sequencing. Raw sequencing data can be analyzed to produce the number of reads, the number of stochastic barcodes with unique molecular label sequences, and the numbers of mRNA molecules.

Disclosed herein include methods for determining the spatial location and copy number of a nucleic acid target in a sample. In some embodiments, the method comprises: providing a substrate comprising a plurality of spatial regions, wherein a plurality of oligonucleotide barcodes are associated with each of the spatial regions, wherein each of the oligonucleotide barcodes comprises a universal sequence, a molecular label, a target-binding region capable of hybridizing to a nucleic acid target, and a predetermined spatial label, wherein oligonucleotide barcodes of the same spatial region comprise the same spatial label, and wherein oligonucleotide barcodes of the different spatial regions comprise different spatial labels. The method can comprise: contacting the substrate with a sample comprising copies of a nucleic acid target, wherein the contacting comprises contacting the plurality of the spatial regions with the sample such that each distinct spatial region contacts a distinct spatial location of the sample. The method can comprise: extending the plurality of oligonucleotide barcodes hybridized to the copies of a nucleic acid target to generate a plurality of barcoded nucleic acid molecules each comprising a sequence complementary to at least a portion of the nucleic acid target. The method can comprise: obtaining sequencing data comprising a plurality of sequencing reads of the plurality of barcoded nucleic acid molecules, or products thereof, wherein each of the plurality of sequencing reads comprises a spatial label sequence, a molecular label sequence, and a subsequence of the nucleic acid target. The method can comprise: for each unique spatial label sequence, which is associated a distinct spatial region of the substrate and thereby a distinct spatial location of the sample, counting the number of molecular labels with distinct sequences associated with a nucleic acid target to determine the copy number of the nucleic acid target at each spatial location of the sample.

Disclosed herein include methods for determining the spatial location and copy number of cellular component targets in a sample. The method can comprise: contacting a plurality of cellular component-binding reagents with the sample, wherein each of the plurality of cellular component-binding reagents comprises a cellular component-binding reagent specific oligonucleotide comprising a unique identifier sequence for the cellular component-binding reagent, and wherein the cellular component-binding reagent is capable of specifically binding to at least one of the plurality of cellular component targets. The method can comprise: extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence. The method can comprise: obtaining sequencing data comprising a plurality of sequencing reads of the plurality of barcoded cellular component-binding reagent specific oligonucleotides, or products thereof, wherein each of the plurality of sequencing reads comprises a spatial label sequence, a molecular label sequence, and at least a portion of the unique identifier sequence. The method can comprise: for each unique spatial label sequence, which is associated a distinct spatial region of the substrate and thereby a distinct spatial location of the sample, counting the number of molecular labels with distinct sequences associated with a unique identifier sequence to determine the number of copies of at least one cellular component target of the plurality of cellular component targets at each spatial location of the sample.

Disclosed herein include compositions. In some embodiments, the composition comprises: a micro-well array comprising a plurality of spatial regions, wherein the micro-well array comprises at least 100 micro-wells, wherein each micro-well has a volume ranging from about 10 $\mu m^3$ to about 786,000 $\mu m^3$, wherein each micro-well comprises a distinct spatial region, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

Disclosed herein include compositions. In some embodiments, the composition comprises: an oligonucleotide array comprising a plurality of spatial regions, wherein the oligonucleotide array comprises at least 100 spots, wherein each spot of the oligonucleotide array comprises a distinct spatial region of the plurality of spatial regions, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adaptors can be linear. The adaptors can be pre-adenylated adaptors. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adaptor can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adaptors can comprise identical and/or universal nucleic acid sequences and the 3' adaptors can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adaptors (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, a "complementary" sequence can refer to a "complement" or a "reverse complement" of a sequence. It is understood from the disclosure that if a molecule can hybridize to another molecule it may be complementary, or partially complementary, to the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene ($-CH_2$), group bridging the 2' oxygen atom and the 4' carbon atom wherein n is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transcriptases, and group II intron reverse transcriptases. Examples of group II intron reverse transcriptases include the *Lactococcus lactis* LI.LtrB intron reverse transcriptase, the *Thermosynechococcus* elongatus Tel4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adaptor primer," "universal primer adaptor" or "universal adaptor sequence" are used interchangeably to refer to a nucleotide sequence that can be used to hybridize to barcodes (e.g., stochastic barcodes) to generate gene-specific barcodes. A universal adaptor sequence can, for example, be a known sequence that is universal across all barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a barcode. For example, a universal adaptor sequence may be part of an oligonucleotide comprising a target-specific sequence to generate a nucleotide sequence that is complementary to a target nucleic acid. A second oligonucleotide comprising a barcode and a complementary sequence of the universal adaptor sequence may hybridize with the nucleotide sequence and generate a target-specific barcode (e.g., a target-specific stochastic barcode). In some embodiments, a universal adaptor primer has a sequence that is different from a universal PCR primer used in the methods of this disclosure.

Barcodes

Barcoding, such as stochastic barcoding, has been described in, for example, Fu et al., Proc Natl Acad Sci U.S.A., 2011 May 31,108(22):9026-31; U.S. Patent Application Publication No. US2011/0160078; Fan et al., Science, 2015 February 6, 347(6222):1258367; US Patent Application Publication No. US2015/0299784; and PCT Application Publication No. WO2015/031691; the content of each of these, including any supporting or supplemental information or material, is incorporated herein by reference in its entirety. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

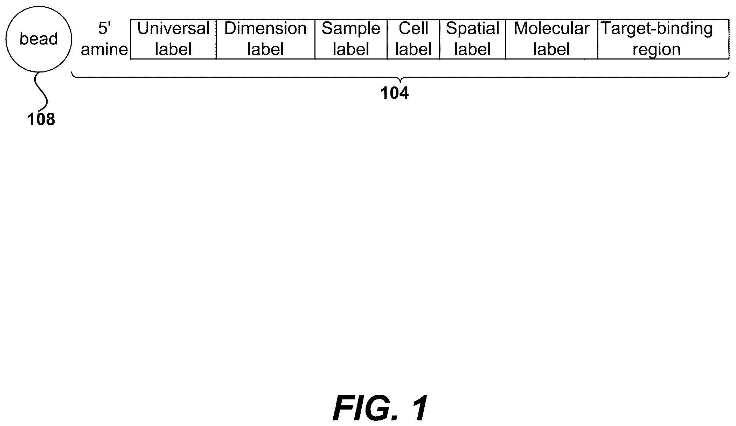

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 105. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

*Universal Labels*

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the barcode to be cleaved off from the support.

*Dimension Labels*

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the GO phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. The dimension label can be reversibly activatable, for example, at least 1, 2, 3, 4, 5, 6, 7, 8, 9,, 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label. In some embodiments, at least 60% of barcodes on the same solid support can comprise the same dimension label. In some embodiments, at least 95% of barcodes on the same solid support can comprise the same dimension label.

There can be as many as $10^6$ or more unique dimension label sequences represented in a plurality of solid supports (e.g., beads). A dimension label can be, or be about 1, 2, 3, 4, 5, 10, 15, 20, 25, 30, 35, 40, 45, 50, or a number or a range between any two of these values, nucleotides in length. A dimension label can be at least, or be at most, 1, 2, 3, 4, 5, 10, 15, 20, 25, 30, 35, 40, 45, 50, 100, 200, or 300, nucleotides in length. A dimension label can comprise between about 5 to about 200 nucleotides. A dimension label can comprise between about 10 to about 150 nucleotides. A dimension label can comprise between about 20 to about 125 nucleotides in length.

*Spatial Labels*

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., A well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, or be about, 60%, 70%, 80%, 85%, 90%, 95%, 97%, 99%, 100%, or a number or a range between any two of these values. In some embodiments, the percentage of barcodes on the same solid support comprising the same spatial label can be at least, or be at most, 60%, 70%, 80%, 85%, 90%, 95%, 97%, 99%, or 100%. In some embodiments, at least 60% of barcodes on the same solid support can comprise the same spatial label. In some embodiments, at least 95% of barcodes on the same solid support can comprise the same spatial label.

There can be as many as $10^6$ or more unique spatial label sequences represented in a plurality of solid supports (e.g., beads). A spatial label can be, or be about, 1, 2, 3, 4, 5, 10, 15, 20, 25, 30, 35, 40, 45, 50, or a number or a range between any two of these values, nucleotides in length. A spatial label can be at least or at most 1, 2, 3, 4, 5, 10, 15, 20, 25, 30, 35, 40, 45, 50, 100, 200, or 300 nucleotides in length. A spatial label can comprise between about 5 to about 200 nucleotides. A spatial label can comprise between about 10 to about 150 nucleotides. A spatial label can comprise between about 20 to about 125 nucleotides in length.

### Cell labels

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all barcodes attached to a given solid support (e.g., a bead), but different for different solid supports (e.g., beads). In some embodiments, the percentage of barcodes on the same solid support comprising the same cell label can be, or be about 60%, 70%, 80%, 85%, 90%, 95%, 97%, 99%, 100%, or a number or a range between any two of these values. In some embodiments, the percentage of barcodes on the same solid support comprising the same cell label can be, or be about 60%, 70%, 80%, 85%, 90%, 95%, 97%, 99%, or 100%. For example, at least 60% of barcodes on the same solid support can comprise the same cell label. As another example, at least 95% of barcodes on the same solid support can comprise the same cell label.

There can be as many as $10^6$ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or be at most, 1, 2, 3, 4, 5, 10, 15, 20, 25, 30, 35, 40, 45, 50, 100, 200, or 300 nucleotides in length. For example, a cell label can comprise between about 5 to about 200 nucleotides. As another example, a cell label can comprise between about 10 to about 150 nucleotides. As yet another example, a cell label can comprise between about 20 to about 125 nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, $10^2$, $10^3$, $10^4$, $10^5$, $10^6$, $10^7$, $10^8$, $10^9$, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, $10^2$, $10^3$, $10^4$, $10^5$, $10^6$, $10^7$, $10^8$, or $10^9$, unique barcode sequences. The unique molecular label sequences can be attached to a given solid support (e.g., a bead). In some embodiments, the unique molecular label sequence is partially or entirely encompassed by a particle (e.g., a hydrogel bead).

The length of a barcode can be different in different implementations. For example, a barcode can be, or be about, 1, 2, 3, 4, 5, 10, 15, 20, 25, 30, 35, 40, 45, 50, or a number or a range between any two of these values, nucleotides in length. As another example, a barcode can be at least, or be at most, 1, 2, 3, 4, 5, 10, 15, 20, 25, 30, 35, 40, 45, 50, 100, 200, or 300 nucleotides in length.

### Molecular Labels

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, $10^2$, $10^3$, $10^4$, $10^5$, $10^6$, $10^7$, $10^8$, $10^9$, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, $10^2$, $10^3$, $10^4$, $10^5$, $10^6$, $10^7$, $10^8$, or $10^9$, unique molecular label sequences. Barcodes with unique molecular label sequences can be attached to a given solid support (e.g., a bead).

For barcoding (e.g., stochastic barcoding) using a plurality of stochastic barcodes, the ratio of the number of different molecular label sequences and the number of occurrence of any of the targets can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. In some embodiments, the ratio of the number of different molecular label sequences and the number of occurrence of any of the targets is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1.

A molecular label can be, or be about, 1, 2, 3, 4, 5, 10, 15, 20, 25, 30, 35, 40, 45, 50, or a number or a range between any two of these values, nucleotides in length. A molecular label can be at least, or be at most, 1, 2, 3, 4, 5, 10, 15, 20, 25, 30, 35, 40, 45, 50, 100, 200, or 300 nucleotides in length.

*Target-Binding Region*

A barcode can comprise one or more target binding regions, such as capture probes. In some embodiments, a target-binding region can hybridize with a target of interest. In some embodiments, the target binding regions can comprise a nucleic acid sequence that hybridizes specifically to a target (e.g., target nucleic acid, target molecule, e.g., a cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, a poly(dA) sequence, a poly(dT) sequence, a poly(dG) sequence, a poly(dC) sequence, or a combination thereof. For example, the target binding region can be an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all barcodes attached to a given bead. In some embodiments, the target binding regions for the plurality of barcodes attached to a given bead can comprise two or more different target binding sequences. A target binding region can be, or be about, 5, 10, 15, 20, 25, 30, 35, 40, 45, 50, or a number or a range between any two of these values, nucleotides in length. A target binding region can be at most about 5, 10, 15, 20, 25, 30, 35, 40, 45, 50 or more nucleotides in length. For example, an mRNA molecule can be reverse transcribed using a reverse transcriptase, such as Moloney murine leukemia virus (MMLV) reverse transcriptase, to generate a cDNA molecule with a poly(dC) tail. A barcode can include a target binding region with a poly(dG) tail. Upon base pairing between the poly(dG) tail of the barcode and the poly(dC) tail of the cDNA molecule, the reverse transcriptase switches template strands, from cellular RNA molecule to the barcode, and continues replication to the 5' end of the barcode. By doing so, the resulting cDNA molecule contains the sequence of the barcode (such as the molecular label) on the 3' end of the cDNA molecule.

In some embodiments, a target-binding region can comprise an oligo(dT) which can hybridize with mRNAs comprising polyadenylated ends. A target-binding region can be gene-specific. For example, a target-binding region can be configured to hybridize to a specific region of a target. A target-binding region can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. A target-binding region can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30, nucleotides in length. A target-binding region can be about 5-30 nucleotides in length. When a barcode comprises a gene-specific target-binding region, the barcode can be referred to herein as a gene-specific barcode.

*Orientation Property*

A stochastic barcode (e.g., a stochastic barcode) can comprise one or more orientation properties which can be used to orient (e.g., align) the barcodes. A barcode can comprise a moiety for isoelectric focusing. Different barcodes can comprise different isoelectric focusing points. When these barcodes are introduced to a sample, the sample can undergo isoelectric focusing in order to orient the barcodes into a known way. In this way, the orientation property can be used to develop a known map of barcodes in a sample. Exemplary orientation properties can include, electrophoretic mobility (e.g., based on size of the barcode), isoelectric point, spin, conductivity, and/or self-assembly. For example, barcodes with an orientation property of self-assembly, can self-assemble into a specific orientation (e.g., nucleic acid nanostructure) upon activation.

*Affinity Property*

A barcode (e.g., a stochastic barcode) can comprise one or more affinity properties. For example, a spatial label can comprise an affinity property. An affinity property can include a chemical and/or biological moiety that can facilitate binding of the barcode to another entity (e.g., cell receptor). For example, an affinity property can comprise an antibody, for example, an antibody specific for a specific moiety (e.g., receptor) on a sample. In some embodiments, the antibody can guide the barcode to a specific cell type or molecule. Targets at and/or near the specific cell type or molecule can be labeled (e.g., stochastically labeled). The affinity property can, in some embodiments, provide spatial information in addition to the nucleotide sequence of the spatial label because the antibody can guide the barcode to a specific location. The antibody can be a therapeutic antibody, for example a monoclonal antibody or a polyclonal antibody. The antibody can be humanized or chimeric. The antibody can be a naked antibody or a fusion antibody.

The antibody can be a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

*Universal Adaptor Primer*

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adaptor primer can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 nucleotides in length. A universal adaptor primer can be from 5-30 nucleotides in length.

*Linker*

When a barcode comprises more than one of a type of label (*e.g.,* more than one cell label or more than one barcode sequence, such as one molecular label), the labels may be interspersed with a linker label sequence. A linker label sequence can be at least about 5, 10, 15, 20, 25, 30, 35, 40, 45, 50 or more nucleotides in length. A linker label sequence can be at most about 5, 10, 15, 20, 25, 30, 35, 40, 45, 50 or more nucleotides in length. In some instances, a linker label sequence is 12 nucleotides in length. A linker label sequence can be used to facilitate the synthesis of the barcode. The linker label can comprise an error-correcting (e.g., Hamming) code.

Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label can be, for example, about 5-20 nucleotides long. A barcode sequence can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (San Francisco, CA). In some implementation, a gel bead can comprise a polymer based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be disruptable (e.g., dissolvable, degradable). For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of $Fe_3O_4$ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus can be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with $SiO_2$ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

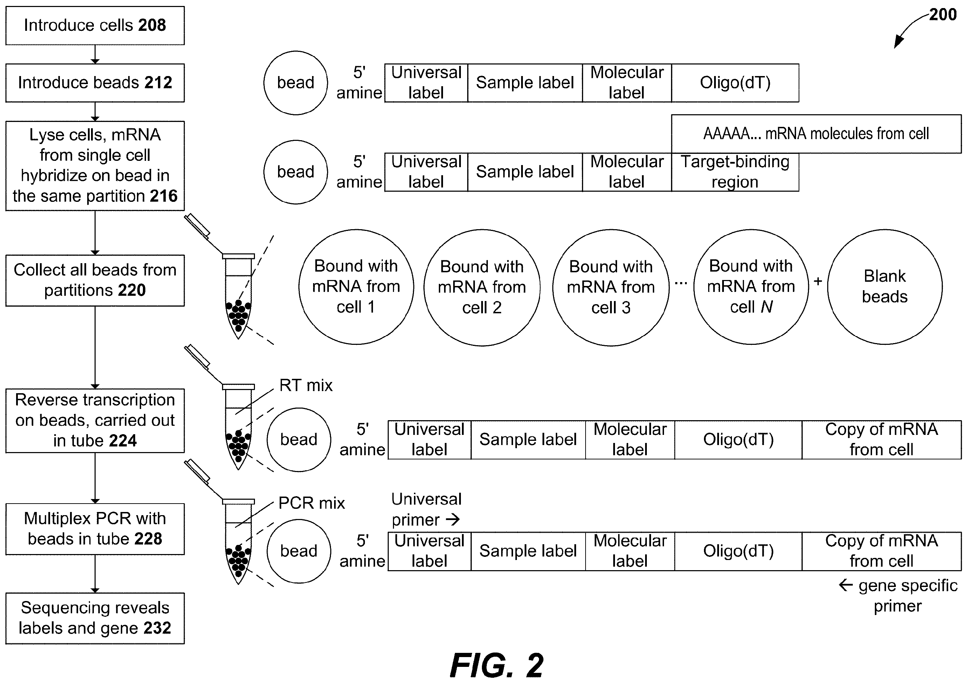

For example, in a non-limiting example of barcoding (e.g., stochastic barcoding) illustrated in FIG. 2, after introducing cells such as single cells onto a plurality of microwells of a microwell array at block 208, beads can be introduced onto the plurality of microwells of the microwell array at block 212. Each microwell can comprise one bead. The beads can comprise a plurality of barcodes. A barcode can comprise a 5' amine region attached to a bead. The barcode can comprise a universal label, a barcode sequence (e.g., a molecular label), a target-binding region, or any combination thereof.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or be at most 60%, 70%, 80%, 85%, 90%, 95%, 97%, 99%, or 100%. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/-Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite ($Fe_3O_4$; magnetite)

nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof.

In some embodiments, the bead (e.g., the bead to which the labels are attached) is a hydrogel bead. In some embodiments, the bead comprises hydrogel.

Some embodiments disclosed herein include one or more particles (for example, beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, $10^6$, $10^7$, $10^8$, $10^9$, a number or a range between any two of these values, or more. In some embodiments, the number of cell label sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, $10^6$, $10^7$, $10^8$, or $10^9$. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more. In some embodiments, none of the plurality of the particles has the same cell label sequence.

The plurality of oligonucleotides on each particle can comprise different barcode sequences (e.g., molecular labels). In some embodiments, the number of barcode sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, $10^6$, $10^7$, $10^8$, $10^9$, or a number or a range between any two of these values. In some embodiments, the number of barcode sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000,

80000, 90000, 100000, $10^6$, $10^7$, $10^8$, or $10^9$. For example, at least 100 of the plurality of oligonucleotides comprise different barcode sequences. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13., 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometer. In some embodiments, the diameter of the bead can be, or be about, 0.1, 0.5, 1, 2, 3, 4, 5, 6, 7, 8, 9, 10, 20, 30, 40, 50 micrometer, or a number or a range between any two of these values.

The diameter of the bead can be related to the diameter of the wells of the substrate. In some embodiments, the diameter of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameter of the bead can be at least, or be at most, 10%, 20%, 30%, 40%, 50%, 60%, 70%, 80%, 90%, or 100% longer or shorter than the diameter of the well. The diameter of the beads can be related to the diameter of a cell (e.g., a single cell entrapped by a well of the substrate). In some embodiments, the diameter of the bead can be, or be about, 10%, 20%, 30%, 40%, 50%, 60%, 70%, 80%, 90%, 100%, 150%, 200%, 250%, 300%, or a number or a range between any two of these values, longer or shorter than the diameter of the cell. In some embodiments, the diameter of the beads can be at least, or be at most, 10%, 20%, 30%, 40%, 50%, 60%, 70%, 80%, 90%, 100%, 150%, 200%, 250%, or 300% longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the barcode on the bead which can serve as a location address.

Examples of beads can include, but are not limited to, streptavidin beads, agarose beads, magnetic beads, Dynabeads®, MACS® microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag™ Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a barcode. A bead can change size, for example, due to swelling in an organic or inorganic solution. A bead can be hydrophobic or hydrophilic. A bead can be biocompatible.

A solid support (e.g., a bead) can be visualized. The solid support can comprise a visualizing tag (e.g., fluorescent dye). A solid support (e.g., a bead) can be etched with an identifier (e.g., a number). The identifier can be visualized through imaging the beads.

A solid support can comprise an insoluble, semi-soluble, or insoluble material. A solid support can be referred to as "functionalized" when it includes a linker, a scaffold, a building block, or other reactive moiety attached thereto, whereas a solid support may be "nonfunctionalized" when it lack such a reactive moiety attached thereto. The solid support can be employed free in solution, such as in a microtiter well format; in a flow-through format, such as in a column; or in a dipstick.

The solid support can comprise a membrane, paper, plastic, coated surface, flat surface, glass, slide, chip, or any combination thereof. A solid support can take the form of resins, gels, microspheres, or other geometric configurations. A solid support can comprise silica chips, microparticles, nanoparticles, plates, arrays, capillaries, flat supports such as glass fiber filters, glass surfaces, metal surfaces (steel, gold silver, aluminum, silicon and copper), glass supports, plastic supports, silicon supports, chips, filters, membranes, microwell plates, slides, plastic materials including multiwell plates or membranes (e.g., formed of polyethylene, polypropylene, polyamide, polyvinylidenedifluoride), and/or wafers, combs, pins or needles (e.g., arrays of pins suitable for combinatorial synthesis or analysis) or beads in an array of pits or nanoliter wells of flat surfaces such as wafers (e.g., silicon wafers), wafers with pits with or without filter bottoms.

The solid support can comprise a polymer matrix (e.g., gel, hydrogel). The polymer matrix may be able to permeate intracellular space (e.g., around organelles). The polymer matrix may able to be pumped throughout the circulatory system.

Substrates and Microwell Array

As used herein, a substrate can refer to a type of solid support. A substrate can refer to a solid support that can comprise barcodes or stochastic barcodes of the disclosure. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., a bead). A microwell can comprise barcode reagents of the disclosure.

Methods of Barcoding

The disclosure provides for methods for estimating the number of distinct targets at distinct locations in a physical sample (e.g., tissue, organ, tumor, cell). The methods can comprise placing barcodes (e.g., stochastic barcodes) in close proximity with the sample, lysing the sample, associating distinct targets with the barcodes, amplifying the targets and/or digitally counting the targets. The method can further comprise analyzing and/or visualizing the information obtained from the spatial labels on the barcodes. In some embodiments, a method comprises visualizing the plurality of targets in the sample. Mapping the plurality of targets onto the map of the sample can include generating a two dimensional map or a three dimensional map of the sample. The two dimensional map and the three dimensional map can be generated prior to or after barcoding (e.g., stochastically barcoding) the plurality of targets in the sample. Visualizing the plurality of targets in the sample can include mapping the plurality of targets onto a map of the sample. Mapping the plurality of targets onto the map of the sample can include generating a two dimensional map or a three dimensional map of the sample. The two dimensional map and the three dimensional map can be generated prior to or after barcoding the plurality of targets in the sample. in some embodiments, the two dimensional map and the three dimensional map can be generated before or after lysing the sample. Lysing the sample before or after generating the two dimensional map or the three dimensional map can include heating the sample, contacting the sample with a detergent, changing the pH of the sample, or any combination thereof.

In some embodiments, barcoding the plurality of targets comprises hybridizing a plurality of barcodes with a plurality of targets to create barcoded targets (e.g., stochastically barcoded targets). Barcoding the plurality of targets can comprise generating an indexed library of the barcoded targets. Generating an indexed library of the barcoded targets can be performed with a solid support comprising the plurality of barcodes (e.g., stochastic barcodes).

*Contacting a Sample and a Barcode*

The disclosure provides for methods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be one-dimensional (e.g., formsa planar surface). The sample (e.g., cells) can be spread across the substrate, for example, by growing/culturing the cells on the substrate.

When barcodes are in close proximity to targets, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

*Cell Lysis*

Following the distribution of cells and barcodes, the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9, 10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7%, or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

*Attachment of Barcodes to Target Nucleic Acid Molecules*

Following lysis of the cells and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of barcoding illustrated in FIG. 2, at block 216, mRNA molecules can hybridize to barcodes on beads. For example, single-stranded nucleotide fragments can hybridize to the target-binding regions of barcodes.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

For example, in a non-limiting example of barcoding illustrated in FIG. 2, at block 220, the labeled targets from a plurality of cells (or a plurality of samples) (e.g., target-barcode molecules) can be subsequently pooled, for example, into a tube. The labeled targets can be pooled by, for example, retrieving the barcodes and/or the beads to which the target-barcode molecules are attached.

The retrieval of solid support-based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

*Reverse Transcription or Nucleic Acid Extension*

The disclosure provides for a method to create a target-barcode conjugate using reverse transcription (e.g., at block 224 of FIG. 2) or nucleic acid extension.

The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of an mRNA molecule to a labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, a target is a cDNA molecule. For example, an mRNA molecule can be reverse transcribed using a reverse transcriptase, such as Moloney murine leukemia virus (MMLV) reverse transcriptase, to generate a cDNA molecule with a poly(dC) tail. A barcode can include a target binding region with a poly(dG) tail. Upon base pairing between the poly(dG) tail of the barcode and the poly(dC) tail of the cDNA molecule, the reverse transcriptase switches template strands, from cellular RNA molecule to the barcode, and continues replication to the 5' end of the barcode. By doing so, the resulting cDNA molecule contains the sequence of the barcode (such as the molecular label) on the 3' end of the cDNA molecule.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

*Amplification*

One or more nucleic acid amplification reactions (e.g., at block 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of labeled targets (e.g., stochastically labeled targets). The one or more primers can anneal to the 3' end or 5' end of the plurality of labeled targets. The one or more primers can anneal to an internal region of the plurality of labeled targets. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

The methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids of the present invention can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at block 232 of FIG. 2.

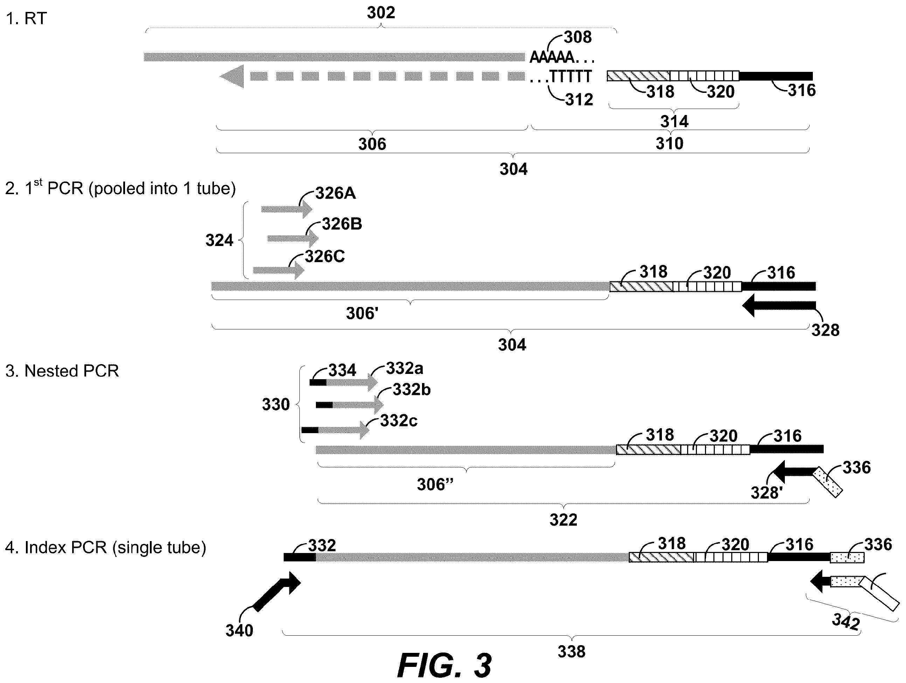

FIG. 3 is a schematic illustration showing a non-limiting exemplary process of generating an indexed library of the barcoded targets (e.g., stochastically barcoded targets), such as barcoded mRNAs or fragments thereof. As shown in step 1, the reverse transcription process can encode each mRNA molecule with a unique molecular label sequence, a cell label sequence, and a universal PCR site. In particular, RNA molecules 302 can be reverse transcribed to produce labeled cDNA molecules 304, including a cDNA region 306, by hybridization (e.g., stochastic hybridization) of a set of barcodes (e.g., stochastic barcodes) 310 to the poly(A) tail region 308 of the RNA molecules 302. Each of the barcodes 310 can comprise a target-binding region, for example a poly(dT) region 312, a label region 314 (e.g., a barcode sequence or a molecule), and a universal PCR region 316.

In some embodiments, the cell label sequence can include 3 to 20 nucleotides. In some embodiments, the molecular label sequence can include 3 to 20 nucleotides. In some embodiments, each of the plurality of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a barcode sequence or a molecular label 318 and a cell label 320. In some embodiments, the label region 314 can include one or more of a universal label, a dimension label, and a cell label. The barcode sequence or molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a barcode sequence or a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of barcodes or stochastic barcodes 310 can contain, contain about, contain at least, or can be at most, 10, 20, 40, 50, 70, 80, 90, $10^2$, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{20}$, or a number or a range between any of these values, barcodes or stochastic barcodes 310. And the set of barcodes or stochastic barcodes 310 can, for example, each contain a unique label region 314. The labeled cDNA molecules 304 can be purified to remove excess barcodes or stochastic barcodes 310. Purification can comprise Ampure bead purification.

As shown in step 2, products from the reverse transcription process in step 1 can be pooled into 1 tube and PCR amplified with a 1st PCR primer pool and a 1st universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, $10^2$, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{20}$, or a number or a range between any of these values, multiplex primers in a single reaction volume. Amplification can comprise using a 1st PCR primer pool

324 comprising custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a 2nd universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a 2nd universal PCR primer 328' in a single reaction volume. The nested PCR primer pool 328 can contain, contain about, contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306" of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the 2nd universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of barcodes or stochastic barcodes 310 can be sequenced in one sequencing reaction using next generation sequencing (NGS).

Spatial Multiomics Methods and Compositions

There are provided, in some embodiments, methods, compositions, systems, and kits for performing spatial multiomics (e.g., a spatial mRNA and protein copier). The methods and compositions can comprise, in some embodiments, in situ reverse transcription using spatial labeled oligonucleotides provided herein.

Disclosed herein include methods and compositions for performing spatial transcriptomics experiments. Currently available spatial transcriptomics methods involve mRNA release from the tissue sections or the use of in situ probe with cleavable barcode (which is subsequently sequenced to obtain spatial mRNA information). The spatial transcriptomics embodiments provided herein differ from both of these approaches and thus do not suffer from their associated deficiencies. Instead of releasing mRNA from the fixed tissue sections, methods provided herein can comprise, in some embodiments, spatially fixed primers binding RNA and performing reverse transcription to copy the mRNA sequence into cDNA using oligonucleotides comprising a spatial barcode. Therefore, in some embodiments, the sections will keep the structure even after the reverse transcription step to copy the information, and can be used for immunostaining afterward. Then, in some embodiments, the immunostaining information can be combined with the sequencing data for comparison and analysis as described herein. Currently available methods wherein RNA is released from tissues and captured suffer from diffusion risk. Currently available methods employing an in situ probe with cleavable barcode and barcode sequencing do not obtain actual sequence information regarding the targets (e.g., mutations).

In the aforementioned currently available method, when RNA is released from the tissue section, there is a diffusion risk which limits the resolution of the spatial information. Additionally, said method needs a space between each location to prevent the diffusion noise. However, in some embodiments of the methods and compositions provided herein, this diffusion risk is reduced or abrogated, as there is not a release of the mRNA out of the tissue and the oligonucleotides comprising the spatial label are physically linked in space to capture only local mRNA around the spatial region. Additionally, the sections can stay intact for other applications (e.g., immunostaining, histology and RNA scope) after RNA copying is done. The methods and compositions provided herein advantageously enable sequencing the RNAs instead of the cleavable barcode (as done in currently available methods). Therefore, non-specific barcode problems suffered by prior art methods due not pose issues in some methods and compositions provided herein. Moreover, due to the low sensitivity of said prior art assays, they do not provide high resolution data either. In contrast, the methods and compositions provided herein can achieve high resolution. Advantageously, in some embodiments, RNA will stay fixed as it can be and just used as a template for reverse transcription, with little or no diffusion risk. Advantageously, in some embodiments, sections will stay as it is so can be used for IF/IHC later without issue. Advantageously, in some embodiments, sections can subsequently be used for in situ analysis if subcellular location of RNA is of interest to a user. Advantageously, in situations where a user desires to sequence only a small region of the section, hybridization/pull down methods provided herein can capture an intended spatial label containing libraries for sequencing, thereby reducing sequencing costs. Advantageously, in some embodiments, since the detection is through sequencing, not through imaging, there is no need to repeat in situ probing/detection.

Without being bound by any particular theory, in some embodiments, in situ reverse transcription can operate on sections. In some embodiments, reverse transcription can be done in fixed cells (e.g., Split-seq). In some embodiments, sequencing can be done in sections (e.g., Readcoor). Without being bound by any particular theory, in some embodiments, the targeted probe can bind to the RNA specifically, and this can be addressed by sequencing result (probe - sequence pairing). In some embodiments, the target-binding region comprises a randomer. In some embodiments, the target-binding region is specific to one or more desired targets. In some embodiments, undesirable species (e.g., genomic DNAs, rRNAs) are blocked using the methods and compositions provided herein. In some embodiments, undesirable species are blocked with decoy oligonucleotides (e.g., with extension blocker). Without being bound by any particular theory, in some embodiments, RNA from fixed sample will not diffuse out - if they are stably in the fixed condition during in situ, it can be same during reverse transcription.

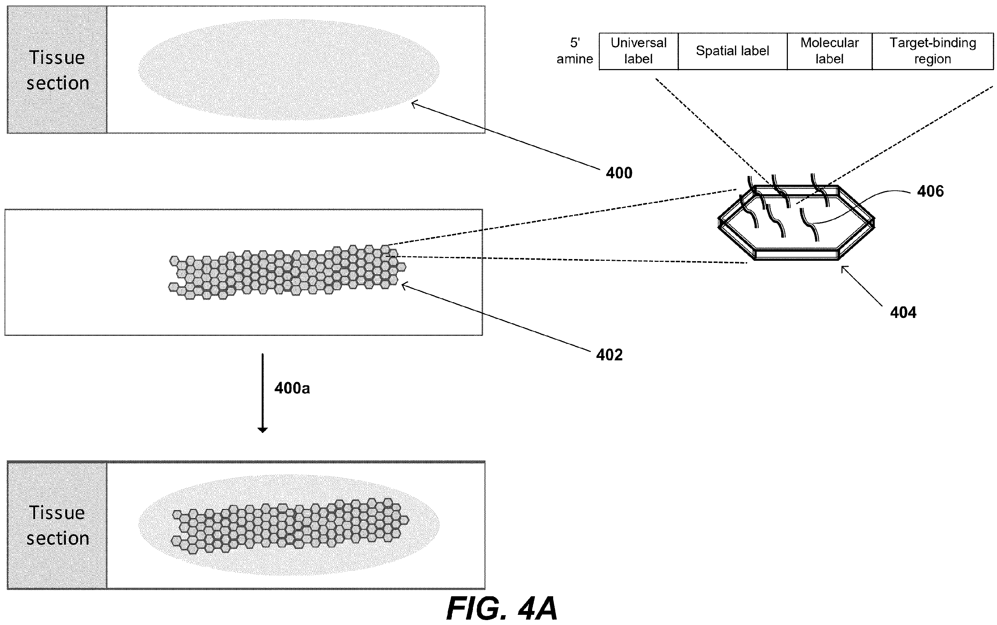

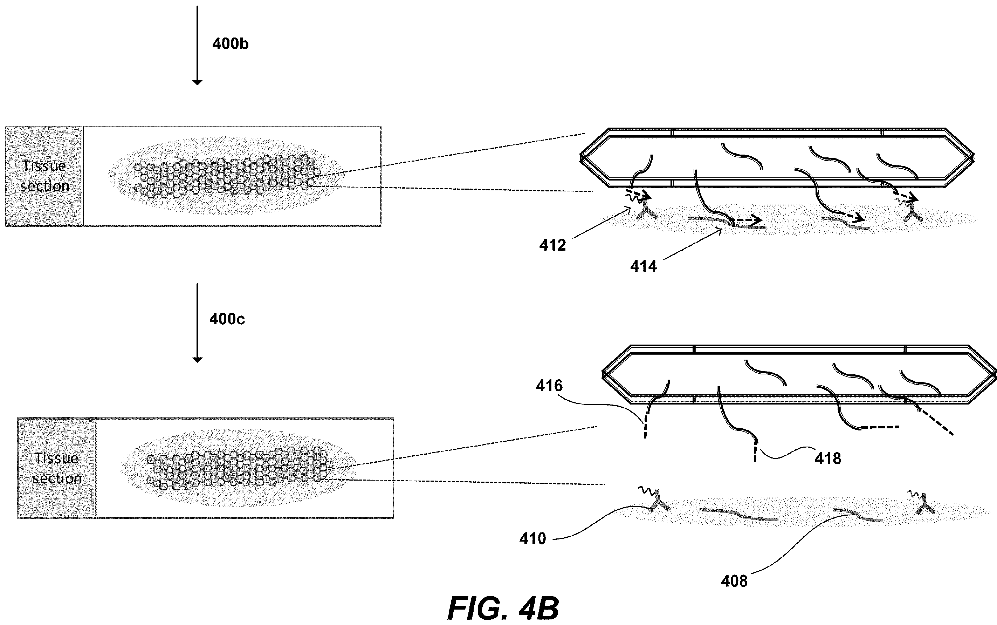

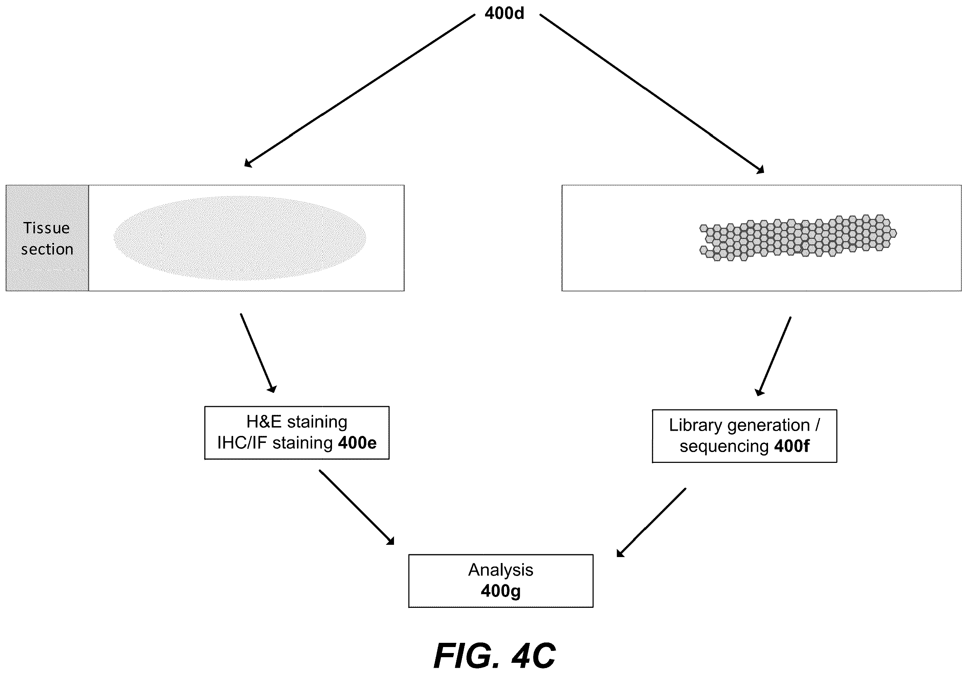

FIGS. 4A-4C depict a non-limiting exemplary spatial multiomics workflow disclosed herein. A sample (e.g., tissue section **400**) can be contacted with a substrate (e.g., micro-well array **402**) at step **400a** of the workflow. Contacting can comprise overlay of the substrate on top of the tissue section with RT reagents. A micro-well array **402** can comprise a plurality of spatial regions. In some embodiments, the micro-well array comprises at least 100 micro-wells **404,** each micro-well has a volume ranging from about 10 $\mu m^3$ to about 786,000 $\mu m^3$, and each micro-well comprises a distinct spatial region. In some embodiments, a plurality of oligonucleotide barcodes **406** are associated with each of the spatial regions (e.g., microwells **404**). Oligonucleotide barcodes **406** can comprise a universal sequence, a molecular label, a target-binding region, and a predetermined spatial label. In some embodiments, oligonucleotide barcodes **406** of the same spatial region comprise the same spatial label, and oligonucleotide barcodes **406** of the different spatial regions comprise different spatial labels. In some embodiments, each well **404** has different spatial label. In some embodiments, the spatial label is pre-set to the well location (spatial region) - it is predetermined. In some embodiments the target-binding region is dT while in other embodiments the plurality of oligonucleotide barcodes comprise a targeted probe set. The oligonucleotide barcodes can be associated with the substrate (e.g., via 5' amine). The sample can comprise nucleic acid targets **408** (e.g., mRNA). The sample can comprise cellular component targets. The workflow can comprise contacting a plurality of cellular component-binding reagents **410** with the sample. Each of the plurality of cellular component-binding reagents **410** can comprise a cellular component-binding reagent specific oligonucleotide comprising a unique identifier sequence for the cellular component-binding reagent. The cellular component-binding reagent **410** can be capable of specifically binding to at least one of the plurality of cellular component targets. The workflow can comprise in situ reverse transcription. The workflow can comprise extending oligonucleotide barcodes hybridized to the cellular component-binding reagent specific oligonucleotides **412** and/or oligonucleotide barcodes hybridized to nucleic acid targets **414** (step **400b),** thereby generating barcoded cellular component-binding reagent specific oligonucleotides **416** and barcoded nucleic acid molecules **418**. The work flow can comprise denaturing off the oligonucleotides (step **400c**). The work flow can comprise physical separation of the sample and the substrate (step **400d**). The work flow can comprise imaging (e.g., H&E staining and/or IHC/IF staining) of the sample (step **400e**). The work flow can comprise library generation and/or sequencing of the barcoded cellular component-binding reagent specific oligonucleotides **416** and barcoded nucleic acid molecules **418,** or products thereof (step **400f**). The work flow can comprise analysis of the imaging and sequencing data (step **400g).** Some embodiments of the methods and compositions provided herein comprise further analysis of the sample after the contacting step. For example, in some embodiments, one or more cells (e.g., single cells) of the sample are lysed and analyzed as described herein.

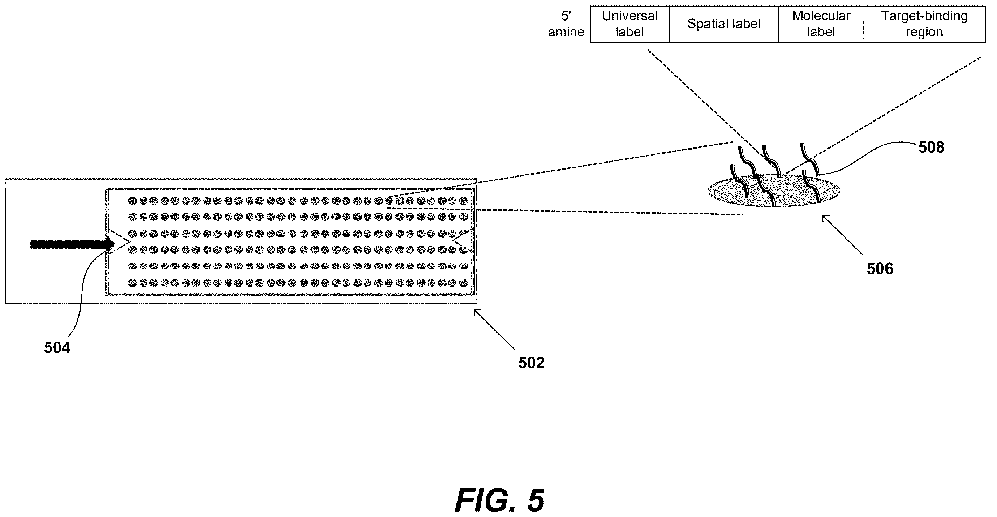

FIG. 5. depicts a non-limiting exemplary spatial multiomics platform. A substrate (e.g., an oligonucleotide array **502**) can comprise a plurality of spatial regions. The oligonucleotide array can comprise at least 100 spots **506.** Each spot **506** of the oligonucleotide array **502** can comprise a distinct spatial region of the plurality of spatial regions. A plurality of oligonucleotide barcodes **508** can be associated with each of the spatial regions. Each of the oligonucleotide barcodes can comprise a universal sequence, a molecular label, a target-binding region, and a predetermined spatial label. Oligonucleotide barcodes of the same spatial region can comprise the same spatial label, and oligonucleotide barcodes of the different spatial regions can comprise different spatial labels. In some embodiments, the array, alone or as part of a cartridge and/or flow cell, can comprises at least one of: an inlet port, an outlet port, a pump, a valve, a vent, a reservoir, a sample collection chamber, a temperature control apparatus, or any combination thereof. The substrate can comprise a buffer input slot **504** for buffer exchange or addition. In some embodiments, there is no divider in the substrate (e.g., well walls), but due to oligonucleotide length, the oligonucleotide binding will be limited in local area.

The methods and systems described herein can be used with methods and systems using antibodies associated with (e.g., attached to or conjugated with) oligonucleotides (also referred to herein as AbOs or AbOligos). Some embodiments of using AbOs to determine protein expression profiles in single cells and tracking sample origins have been described in US2018/0088112, and US2018/0346970; the content of each is incorporated by reference herein in its entirety. In some embodiments, the method disclosed herein allows V(D)J profiling of T cells and B cells, 3' targeted, 5' targeted, 3' whole transcriptome amplification (WTA), 5' WTA, protein expression profiling with AbO, and/or sample multiplexing on a single experiment. Methods for determining the sequences of a nucleic acid target (e.g., the V(D)J region of an immune receptor) using 5' barcoding and/or 3' barcoding are described in US2020/0109437; the content of which is incorporated herein by reference in its entirety. Systems, methods, compositions, and kits for molecular barcoding on the 5'-end of a nucleic acid target have been described in, for example, US2019/0338278, the content of which is incorporated herein by reference in its entirety. The systems, methods, compositions, and kits for 5'-based gene expression profiling provided herein can, in some embodiments, be employed in concert with the methods to obtain full-length V(D)J information (e.g., by Illumina sequencing on the Rhapsody system) using a combined 5' barcoding and random priming approach described in U.S. Patent Application Number 17/091,639, the content of which is incorporated herein by reference in its entirety. The systems, methods, compositions, and kits for 5'-based gene expression profiling provided herein can, in some embodiments, be employed in concert with random priming and extension (RPE)-based whole transcriptome analysis methods and compositions have been described in U.S. Patent Application No. 16/677,012; the content of which is incorporated herein by reference in its entirety.

Disclosed herein include methods for determining the spatial location and copy number of a nucleic acid target in a sample. In some embodiments, the method comprises: providing a substrate comprising a plurality of spatial regions, wherein a plurality of oligonucleotide barcodes are associated with each of the spatial regions, wherein each of the oligonucleotide barcodes comprises a universal sequence, a molecular label, a target-binding region capable of hybridizing to a nucleic acid target, and a predetermined spatial label, wherein oligonucleotide barcodes of the same spatial region comprise the same spatial label, and wherein oligonucleotide barcodes of the different spatial regions comprise different spatial labels. The method can comprise: contacting the substrate with a sample comprising copies of a nucleic acid target, wherein the contacting comprises contacting the plurality of the spatial regions with the sample such that each distinct spatial region contacts a distinct spatial location of the sample. The method can comprise: extending the plurality of oligonucleotide barcodes hybridized to the copies of a nucleic acid target to generate a plurality of barcoded nucleic acid molecules each comprising a sequence complementary to at least a portion of the nucleic acid target. The method can comprise: obtaining sequencing data comprising a plurality of sequencing reads of the plurality of barcoded nucleic acid molecules, or products thereof, wherein each of the plurality of sequencing reads comprises a spatial label sequence, a molecular label sequence, and a subsequence of the nucleic acid target. The method can comprise: for each unique spatial label sequence, which is associated a distinct spatial region of the substrate and thereby a distinct spatial location of the sample, counting the number of molecular labels with distinct sequences associated with a nucleic acid target to determine the copy number of the nucleic acid target at each spatial location of the sample.

Disclosed herein include methods for determining the spatial location and copy number of cellular component targets in a sample. The method can comprise: contacting a plurality of cellular component-binding reagents with the sample, wherein each of the plurality of cellular component-binding reagents comprises a cellular component-binding reagent specific oligonucleotide comprising a unique identifier sequence for the cellular component-binding reagent, and wherein the cellular component-binding reagent is capable of specifically binding to at least one of the plurality of cellular component targets. The method can comprise: extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence. The method can comprise: obtaining sequencing data comprising a plurality of sequencing reads of the plurality of barcoded cellular component-binding reagent specific oligonucleotides, or products thereof, wherein each of the plurality of sequencing reads comprises a spatial label sequence, a molecular label sequence, and at least a portion of the unique identifier sequence. The method can comprise: for each unique spatial label sequence, which is associated a distinct spatial region of the substrate and thereby a distinct spatial location of the sample, counting the number of molecular labels with distinct sequences associated with a unique identifier sequence to determine the number of copies of at least one cellular component target of the plurality of cellular component targets at each spatial location of the sample.

In some embodiments, at least a portion of the contacting step is performed in the presence in the presence of extension reagents, optionally the entire contacting step is performed in the presence in the presence of the extension reagents, further optionally the contacting and extension steps are simultaneous, optionally the sample and substrate are in contact during the extension step, optionally the extension is performed in situ. In some embodiments, the extension reagents comprise reverse transcription reagents, optionally reverse transcription reagents comprise a reverse transcriptase and dNTPs. In some embodiments, the method comprises a denaturing step, optionally denaturing separates the nucleic acid target from the barcoded nucleic acid molecule and/or the cellular component-binding reagent specific oligonucleotide from the barcoded cellular component-binding reagent specific oligonucleotide. In some embodiments, the contacting step comprises an overlay of the substrate on the sample. The method can comprise: separating the substrate from the sample from the substrate after the contacting step. In some embodiments, the sample is contacted with a fixative and/or permeabilizing agent before, during, and/or after the contacting step. In some embodiments, the sample is not contacted with a fixative and/or permeabilizing agent before, during, and/or after the contacting step.

In some embodiments, the method (i) comprises fixing the sample prior to the contacting step or (ii) does not comprise fixing the sample prior to the contacting step. In some embodiments, the sample is physically divided or is intact during the contacting step. In some embodiments, the spatial locations of the nucleic acid targets in the sample are on a surface of the sample, inside the sample, subcellularly in the sample, or any combination thereof. In some embodiments, the sample comprises a plurality of cells, optionally the nucleic acid targets are associated with the plurality of cells. In some embodiments, the plurality of cells comprise one or more cell types, optionally said one or more cell types are selected from the group consisting of: brain cells, heart cells, cancer cells, circulating tumor cells, organ cells, epithelial cells, metastatic cells, benign cells, primary cells, and circulatory cells, or any combination thereof. In some embodiments, the sample comprises a tissue, a cell monolayer, fixed cells, a tissue section, or any combination thereof. In some embodiments, the sample comprises a biological sample, a clinical sample, an environmental sample, a biological fluid, a tissue, a tissue section derived from a subject, or any combination thereof, optionally the subject is a human, a mouse, a dog, a rat, or a vertebrate.

The method can comprise: determining genotype, phenotype, or one or more genetic mutations of the subject based on the spatial location of the nucleic acid targets in the sample. The method can comprise: predicting susceptibility of the subject to one or more diseases. In some embodiments, at least one of the one or more diseases is cancer or a hereditary disease. The method can comprise: determining cell types of the plurality of cells in the sample. In some embodiments, a drug is chosen based on predicted responsiveness of the cell types of the plurality of cells in the sample. The method can comprise: isolating one or more barcoded nucleic acid molecules (or products thereof) corresponding to a spatial location of interest, optionally the isolating comprises selective hybridization and pull-down of one or more barcoded nucleic acid molecules comprising the unique spatial label of a spatial region corresponding to a spatial location of interest.

In some embodiments, the oligonucleotide barcode comprises a target-binding region comprising a capture sequence, optionally the target-binding region comprises a poly(dT) region. In some embodiments, the cellular component-binding reagent specific oligonucleotide comprises a sequence complementary to the capture sequence configured to capture the cellular component-binding reagent specific oligonucleotide, optionally the sequence complementary to the capture sequence comprises a poly(dA) region. In some embodiments, the nucleic acid target comprises a nucleic acid molecule. In some embodiments, the nucleic acid molecule comprises ribonucleic acid (RNA), messenger RNA (mRNA), microRNA, small interfering RNA (siRNA), RNA degradation product, RNA comprising a poly(A) tail, a sample indexing oligonucleotide, a cellular component-binding reagent specific oligonucleotide, or any combination thereof. In some embodiments, each of the spatial regions comprises a plurality of oligonucleotide barcodes configured to hybridize to a panel of nucleic acid targets. In some embodiments, the cellular component-binding reagent specific oligonucleotide comprises a second universal sequence. In some embodiments, obtaining sequence information of the plurality of barcoded cellular component-binding reagent specific oligonucleotides, or products thereof, comprises: amplifying the plurality of barcoded cellular component-binding reagent specific oligonucleotides, or products thereof, using a primer capable of hybridizing to the first universal sequence, or a complement thereof, and a primer capable of hybridizing to the second universal sequence, or a complement thereof, to generate a plurality of amplified barcoded cellular component-binding reagent specific oligonucleotides; and obtaining sequencing data of the plurality of amplified barcoded cellular component-binding reagent specific oligonucleotides, or products thereof. In some embodiments, obtaining sequencing data comprises attaching sequencing adaptors to (i) the plurality of barcoded nucleic acid molecules, or products thereor, and/or (ii) the plurality of barcoded cellular component-binding reagent specific oligonucleotides, or products thereof.

The method can comprise: after contacting the plurality of cellular component-binding reagents with the sample, removing one or more cellular component-binding reagents of the plurality of cellular component-binding reagents that are not contacted with the sample, optionally removing the one or more cellular component-binding reagents not contacted with the sample comprises: removing the one or more cellular component-binding reagents not contacted with the respective at least one of the plurality of cellular component targets. In some embodiments, the cellular component target comprises an intracellular protein, a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof.

In some embodiments, extending the plurality of oligonucleotide barcodes comprises extending the plurality of oligonucleotide barcodes using a reverse transcriptase and/or a DNA polymerase lacking at least one of 5' to 3' exonuclease activity and 3' to 5' exonuclease activity, optionally the DNA polymerase comprises a Klenow Fragment, further optionally the reverse transcriptase comprises a viral reverse transcriptase, optionally wherein the viral reverse transcriptase is a murine leukemia virus (MLV) reverse transcriptase or a Moloney murine leukemia virus (MMLV) reverse transcriptase. In some embodiments, less than about 5 percent of nucleic acid targets are mapped to an incorrect spatial location due to diffusion of said nucleic acid target from a starting spatial location.

In some embodiments, wherein each of the plurality of oligonucleotide barcodes comprises a substrate linker functional group, each of the plurality of substrates comprises a substrate functional group, and the substrate functional group and the substrate linker functional group are associated with each other. In some embodiments, the substrate linker functional group and the substrate functional group are individually selected from the group consisting of C6, biotin, streptavidin, primary amine(s), aldehyde(s), ketone(s), and any combination thereof. In some embodiments, the oligonucleotide barcode is associated with the substrate through a substrate linker. In some embodiments, the substrate linker comprises a carbon chain, optionally the carbon chain comprises 2-30 carbons, and further optionally the carbon chain comprises 12 carbons. In some embodiments, the substrate linker comprises 5' amino modifier C12 (5AmMCl2), or a derivative thereof. In some embodiments, the spatial label is 6-60 nucleotides in length. In some embodiments, the oligonucleotide barcode is 50-500 nucleotides in length. In some embodiments, the oligonucleotide barcode is attached to the substrate. In some embodiments, the oligonucleotide barcode is covalently attached to the substrate. In some embodiments, the oligonucleotide barcode is conjugated to the substrate. In some embodiments, the oligonucleotide barcode is conjugated to the substrate through a chemical group selected from the group consisting of a UV photocleavable group, a streptavidin, a biotin, an amine, and a combination thereof. In some embodiments, the oligonucleotide barcode is non-covalently attached to the substrate.

In some embodiments, the oligonucleotide barcode is configured to be detachable from the substrate, The method can comprise: dissociating the oligonucleotide barcode from the substrate, and optionally dissociating the oligonucleotide barcode from the substrate comprises detaching the oligonucleotide barcode from the substrate by UV photocleaving, chemical treatment, heating, enzyme treatment, or any combination thereof. In some embodiments, the dissociating occurs after barcoding the oligonucleotide barcodes.

In some embodiments, the oligonucleotide barcode is configured to be non-detachable from the substrate. In some embodiments, the oligonucleotide barcode is conjugated to the substrate by a 1,3-dipolar cycloaddition reaction, a hetero-Diels-Alder reaction, a nucleophilic substitution reaction, a non-aldol type carbonyl reaction, an addition to carbon-carbon multiple bond, an oxidation reaction, a click reaction, or any combination thereof. In some embodiments, at least one oligonucleotide barcode of the plurality of oligonucleotide barcodes is immobilized on the substrate, partially immobilized on the substrate, enclosed in the substrate, partially enclosed in the substrate, or a combination thereof. In some embodiments, the substrate comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof. In some embodiments, the target-binding region comprises a gene-specific sequence, an oligo(dT) sequence, a random multimer, or any combination thereof. In some embodiments, each spatial label of the plurality of oligonucleotide barcodes comprise at least 6 nucleotides. In some embodiments, each molecular label of the plurality of oligonucleotide barcodes comprises at least 6 nucleotides.

In some embodiments, the substrate comprises a solid support. In some embodiments, the solid support comprises a planar surface. In some embodiments, the substrate comprises an oligonucleotide array comprising a plurality of spots, and wherein each spot of the oligonucleotide array comprises a distinct spatial region of the plurality of spatial regions. In some embodiments, the substrate comprises a microwell array, and wherein each microwell of the microwell array comprises a distinct spatial region of the plurality of spatial regions.

Imaging

The method can comprise: imaging the sample, optionally imaging the sample after the contacting step, optionally the imaging generates imaging data. In some embodiments, imaging the sample comprises staining the sample with a stain, wherein the stain is a fluorescent stain, a negative stain, an antibody stain, or any combination thereof, optionally staining comprises Immunocytochemistry (ICC), Immunohistochemistry (IHC), Immunofluorescence (IF), or any combination thereof. In some embodiments, imaging comprises microscopy, confocal microscopy, time-lapse imaging microscopy, fluorescence microscopy, multi-photon microscopy, quantitative phase microscopy, surface enhanced Raman spectroscopy, videography, manual visual analysis, automated visual analysis, or any combination thereof.

The sample contacted to the substrate can be analyzed (e.g., with immunohistochemistry, staining and/or imaging). Exemplary methods of immunohistochemistry can comprise a step of reacting a labeled probe biological substance obtained by introducing a label into a substance capable of recognizing a biological substance to be detected to a tissue section, to visualize the biological substance to be detected present on the tissue section via a specific binding reaction between the biological substances.

For histology specimens, the tissue pieces can be fixed in a suitable fixative, typically formalin, and embedded in melted paraffin wax. The wax block can be cut on a microtome to yield a thin slice of paraffin containing the tissue. The specimen slice can be applied to a substrate, air dried, and heated to cause the specimen to adhere to the glass slide. Residual paraffin can be dissolved with a suitable solvent, typically xylene, toluene, or others. These so-called deparaffinizing solvents can be removed with a washing-dehydrating type reagent prior to staining. Slices can be prepared from frozen specimens, fixed briefly in 10% formalin, then infused with dehydrating reagent. The dehydrating reagent can be removed prior to staining with an aqueous stain.

In some embodiments, the Papanicolaou staining technique can be used (e.g., a progressive stain and/or hematoxylineosin $[H\&E]$, i.e., a regressive stain). HE (hematoxylineosin) stain uses hematoxylin and eosin as a dye. Hematoxylin is a blue-violet dye, and has a property of staining basophilic tissues such as cell nuclei, bone tissues, part of cartilage tissues, and serous components. Eosin is a red to pink dye, and has a property of staining eosinophilic tissues such as cytoplasm, connective tissues of the softtissue, red blood cells, fibrin, and endocrine granules.

Immunohistochemistry (IHC) can be referred to as "immunological staining" due to the process of color development for visualizing an antigen-antibody reaction which is otherwise invisible (hereinafter, the term "immunohistochemical staining" can be used for immunohistochemistry). Lectin staining is a technique that can use a property of lectin of binding to a specific sugar chain in a non-immunological and specific manner in order to detect a sugar chain in a tissue specimen using lectin.

HE staining, immunohistochemistry and lectin staining can be used for detecting a location of, for example, cancer cells in a cell specimen. For example, when it is desired to confirm a location of cancer cells in a cell specimen, a pathologist, in order to determine the presence or absence of cancer cells in the cell specimen, can prepare tissue sections and place them on a substrate of the disclosure. The section on the array can be subjected to HE staining, imaging, or any immunohistochemical analysis in order to obtain its morphological information and/or any other identifying features (such as presence or absence of rare cells). The sample can be lysed and the presence or absence of nucleic acid molecules can be determined using the methods of the disclosure. The nucleic acid information can be compared (e.g., spatially compared) to the image, thereby indicating the spatial location of nucleic acids in a sample.

In some embodiments, the tissue is stained with a staining enhancer (e.g., a chemical penetrant enhancer). Examples of tissue chemical penetrant enhancers that facilitate penetration of the stain into the tissue include, but are not limited to, polyethylene glycol (PEG), surfactants such as polyoxyethylenesorbitans, polyoxyethylene ethers (polyoxyethylenesorbitan monolaurate (Tween 20) and other Tween derivatives, polyoxyethylene 23 lauryl ether (Brij 35), Triton X-100, Brij 35, Nonidet P-40, detergent-like substances such as lysolecithins, saponins, non-ionic detergents such as TRITON® X-100, etc., aprotic solvents such as dimethyl sulfoxide (DMSO), ethers such as tetrahydrofuran, dioxane, etc.; esters such as ethyl acetate, butyl acetate, isopropyl acetate; hydrocarbons such as toluene, chlorinated solvents such as dichloromethane, dichloroethane, chlorobenzene, etc.; ketones such as acetone, nitriles such as acetonitrile, and/or other agents that increase cell membrane permeability.

In some embodiments, a composition is provided that facilitates staining of a mammalian tissue sample. The composition can comprise a stain, such as hematoxylin, or hematoxylin and eosin-Y, at least one tissue chemical penetrant enhancer, such as a surfactant, an aprotic solvent, and/or PEG, or any combination thereof.

In some embodiments, the sample is imaged (e.g., either before or after IHC or without IHC). Imaging can comprise microscopy such as bright field imaging, oblique illumination, dark field imaging, dispersion staining, phase contrast, differential interference contrast, interference reflection microscopy, fluorescence, confocal, electron microscopy, transmission electron microscopy, scanning electron microscopy, and single plane illumination, or any combination thereof. Imaging can comprise the use of a negative stain (e.g., nigrosin, ammonium molybdate, uranyl acetate, uranyl formate, phosphotungstic acid, osmium tetroxide). Imaging can comprise the use of heavy metals (e.g., gold, osmium) that can scatter electrons.

Imaging can comprise imaging a portion of the sample (e.g., slide/array). Imaging can comprise imaging at least 10, 20, 30, 40, 50, 60, 70, 80, 90, or 100% of the sample. Imaging can comprise imaging at most 10, 20, 30, 40, 50, 60, 70, 80, 90, or 100% of the sample. Imaging can be done in discrete steps (e.g., the image may not need to be contiguous). Imaging can comprise taking at least 1, 2, 3, 4, 5, 6, 7, 8, 9, or 10 or more different images. Imaging can comprise taking at most 1, 2, 3, 4, 5, 6, 7, 8, 9, or 10 or more different images.

Analysis of Sequencing Data and Imaging Data

The method can comprise: associating the imaging data and sequencing data of one or more spatial locations of the sample. The method can comprise: correlation analysis of the imaging data and the sequencing data of the spatial locations, optionally the correlation analysis identifies one or more of the following: candidate biomarkers, candidate therapeutic agents, candidate doses of therapeutic agents, and/or cellular targets of candidate therapeutic agents. In some embodiments, said imaging produces an image that is used to construct a map of a physical representation of said sample, optionally said map is two dimensional or three dimensional. The method can comprise: mapping the nucleic acid targets and/or cellular component targets onto the map of the sample.

The data from the substrate scan can be correlated to the image of the unlysed sample on the substrate. The data can be overlayed thereby generating a map. A map of the location of targets from a sample can be constructed using information generated using the methods described herein. The map can be used to locate a physical location of a target. The map can be used to identify the location of multiple targets. The multiple targets can be the same species of target, or the multiple targets can be multiple different targets. For example a map of a brain can be constructed to show the amount and location of multiple targets.

The map can be generated from data from a single sample. The map can be constructed using data from multiple samples, thereby generating a combined map. The map can be constructed with data from tens, hundreds, and/or thousands of samples. A map constructed from multiple samples can show a distribution of targets associated with regions common to the multiple samples. For example, replicated assays can be displayed on the same map. At least 1, 2, 3, 4, 5, 6, 7, 8, 9, or 10 or more replicates can be displayed (e.g., overlaid) on the same map. At most 1, 2, 3, 4, 5, 6, 7, 8, 9, or 10 or more replicates can be displayed (e.g., overlaid) on the same map. The spatial distribution and number of targets can be represented by a variety of statistics.

Combining data from multiple samples can increase the locational resolution of the combined map. The orientation of multiple samples can be registered by common landmarks and/or x-y positions on the array, wherein the individual locational measurements across samples are at least in part non-contiguous. Multiplexing the above approach will allow for high resolution maps of target nucleic acids in a sample.

The data analysis and correlation can be useful for determining the presence and/or absence of a specific cell type (e.g., rare cell, cancer cell). The data correlation can be useful for determining the relative ratios of target nucleic acids in distinct locations either within a cell, or within a sample.

The methods and compositions disclosed herein can be companion diagnostics for a medical professional (e.g., a pathologist) wherein a subject can be diagnosed by visually looking at a pathology image and correlating the image to genetic expression (e.g., identification of expression of oncogenes). The methods and compositions can be useful for identifying a cell from a population of cells, and determining the genetic heterogeneity of the cells within a sample. The methods and compositions can be useful for determining the genotype of a sample.

## Sample Treatment

Some embodiments of the methods and compositions provided herein comprise treatment of the sample with fixing agent(s), unfixing agent(s), and/or permeabilizing agent(s), before, during, and/or after contacting with the substrate.

### Fixing Agents and Unfixing Agents

As described herein, the fixing agent can comprise a cross-linking agent. The fixing agent can comprise a cleavable cross-linking agent. The cleavable cross-linking agent can comprise a thiol-cleavable cross-linking agent. The cleavable cross-linking agent can comprise or can be derived from dithiobis(succinimidyl propionate) (DSP, Lomant's Reagent), disuccinimidyl tartrate (DST), Bis [2-(Succinimidooxycarbonyloxy)ethyl] Sulfone (BSOCOES), ethylene glycol bis(succinimidyl succinate) (EGS), dimethyl 3,3'-dithiobispropionimidate (DTBP, Wang and Richard's Reagent), succinimidyl 3-(2-pyridyldithio)propionate (SPDP), succinimidyl 6-(3(2-pyridyldithio)propionamido)hexanoate (LC-SPDP), 4-succinimidyloxycarbonyl-alpha-methyl-$\alpha$(2-pyridyldithio)toluene (SMPT), 3-(2-pyridyldithio)propionyl hydrazide (PDPH), succinimidyl 2-((4,4'-azipentanamido)ethyl)-1,3'-dithiopropionate (SDAD, NHS-SS-Diazirine), or any combination thereof. The cleavable cross-linking agent can comprise a cleavable linkage selected from the group consisting of a chemically cleavable linkage, a photocleavable linkage, an acid labile linker, a heat sensitive linkage, an enzymatically cleavable linkage, or any combination thereof. The cleavable cross-linking agent can comprise a disulfide linker. The fixing agent can comprise BD Cytofix. The fixing agent can comprise a reversible cross-linker. The fixing agent can comprise a non-cross-linking fixative. The non-cross-linking fixative can comprise methanol. In some embodiments, the fixing agent (i.e., fixative) is or comprises aldehydes, including but not limited to paraformaldehyde (PFA), formaldehyde and glutaraldehyde, or a combination thereof.

Non-limiting examples of fixing agent include, but are not limited to, NHS (N-hydroxysuccinimide); sulfo-NHS (N-hydroxysulfosuccinimide); EDC (1-Ethyl-3-[3-dimethylaminopropyl]); carbodiimide hydrochloride; SMCC (succinimidyl 4-(N-maleimidomethyl)cyclohexane-1-carboxylate); sulfo-SMCC; DSS (di succinimidyl suberate); DSG (disuccinimidyl glutarate); DFDNB (1,5-difluoro-2,4-dinitrobenzene); BS3 (bis(sulfosuccinimidyl)suberate); TSAT (tris-(succinimidyl)aminotriacetate); BS(PEG)5 (PEGylated bis(sulfosuccinimidyl)suberate); BS(PEG)9 (PEGylated bis(sulfosuccinimidyl)-suberate); DSP(dithiobis(succinimidyl propionate)); DTSSP (3,3'-dithiobis(sulfosuccinimidyl propionate)); DST(disuccinimidyl tartrate); BSOCOES (bis(2-(succinimidooxycarbonyloxy)-ethyl)sulfone); EGS (ethylene glycol bis(succinimidyl succinate)); DMA (dimethyl adipimidate); DMP (dimethyl pimelimidate); DMS (dimethyl suberimidate); DTBP (Wang and Richard's Reagent); BM(PEG)2 (1,8-bismaleimido-diethyleneglycol); BM(PEG)3 (1,11-bismaleimido-triethyleneglycol); BMB (1,4-bismaleimidobutane); DTME (dithiobismaleimidoethane); BMH (bismaleimidohexane); BMOE (bismaleimidoethane); TMEA (tris(2-maleimidoethyl)amine); SPDP (succinimidyl 3-(2-pyridyldithio) propionate); SMCC (Succinimidyl trans-4-(maleimidylmethyl)cyclohexane-1-Carboxylate); SIA (succinimidyl iodoacetate); SBAP (succinimidyl 3-(bromoacetamido) propionate); STAB (succinimidyl (4-iodoacetyl)-aminobenzoate); Sulfo-SIAB (sulfosuccinimidyl (4-iodoacetyl) aminobenzoate); AMAS (N-α-maleimidoacet-oxysuccinimide ester); BMPS (N-β-maleimidopropyl-oxysuccinimide ester); GMBS (N-γ-maleimidobutyryl-oxysuccinimide ester); Sulfo-GMBS (N-γ-maleimidobutyryl-oxysulfosuccinimide ester); MBS (m-maleimidobenzoyl-N-hydroxysuccinimide ester); Sulfo-MBS (m-maleimidobenzoyl-N-hydroxysulfosuccinimide ester); SMCC (succinimidyl 4-(N-maleimidomethyl)cyclohexane-1-carboxylate); Sulfo-SMCC (sulfosuccinimidyl 4-(N-maleimidomethyl)cyclohexane-1-carboxylate); EMCS (N-ε-malemidocaproyl-oxysuccinimide ester); Sulfo-EMCS (N-ε-maleimidocaproyl-oxysulfosuccinimide ester); SMPB (succinimidyl 4-(p-maleimidophenyl)butyrate); Sulfo-SMPB (sulfosuccinimidyl 4-(N-maleimidophenyl)-butyrate); SMPH (Succinimidyl 6-((beta-maleimidopropionamido)-hexanoate)); LC-SMCC (succinimidyl 4-(N-maleimidomethyl) cyclohexane-1-carboxy-(6-amidocaproate)), Sulfo-KMUS (N-κ-maleimidoundecanoyl-oxysulfosuccinimide ester); SPDP (succinimidyl 3-(2-pyridyldithio)propionate); LC-SPDP (succinimidyl 6-(3(2-pyridyldithio)propionamido) hexanoate); LC-SPDP (succinimidyl 6-(3(2-pyridyldithio)propionamido) hexanoate); Sulfo-LC-SPDP (sulfosuccinimidyl 6-(3'-(2-pyridyldithio)propionamido)hexanoate); SMPT (4-succinimidyloxycarbonyl-alpha-methyl-α(2-pyridyldithio)toluene); PEG4-SPDP (PEGylated, long-chain SPDP crosslinker); PEG12-SPDP (PEGylated, long-chain SPDP crosslinker); SM(PEG)2 (PEGylated SMCC crosslinker); SM(PEG)4 (PEGylated SMCC crosslinker); SM(PEG)6 (PEGylated, long-chain SMCC crosslinker); SM(PEG)8 (PEGylated, long-chain SMCC crosslinker); SM(PEG)12 (PEGylated, long-chain SMCC crosslinker); SM(PEG)24 (PEGylated, long-chain SMCC crosslinker); BMPH (N-β-maleimidopropionic acid hydrazide); EMCH (N-ε-maleimidocaproic acid hydrazide); MPBH (4-(4-N-maleimidophenyl)butyric acid hydrazide); KMUH (N-x-maleimidoundecanoic acid hydrazide); PDPH (3-(2-pyridyldithio)-propionyl hydrazide); ATFB-SE (4-Azido-2,3,5,6-Tetrafluorobenzoic Acid, Succinimidyl Ester); ANB-NOS (N-5-azido-2-nitrobenzoyloxysuccinimide); SDA (NHS-Diazirine) (succinimidyl 4,4'-azipentanoate);

LC-SDA (NHS-LC-Diazirine) (succinimidyl 6-(4,4'-azipentanamido)hexanoate); SDAD (NHS-SS-Diazirine) (succinimidyl 2-((4,4'-azipentanamido)ethyl)-1,3'-dithiopropionate); Sulfo-SDA (Sulfo-NHS-Diazirine) (sulfosuccinimidyl 4,4'-azipentanoate); Sulfo-LC-SDA (Sulfo-NHS-LC-Diazirine) (sulfosuccinimidyl 6-(4,4'-azipentanamido)hexanoate); Sulfo-SDAD (Sulfo-NHS-SS-Diazirine) (sulfosuccinimidyl 2-((4,4'-azipentanamido) ethyl)-1,3'-dithiopropionate); SPB (succinimidyl-[4-(psoralen-8-yloxy)]-butyrate); Sulfo-SANPAH (sulfosuccinimidyl 6-(4'-azido-2'-nitrophenylamino)hexanoate); DCC (dicyclohexylcarbodiimide); EDC (1-ethyl-3-(3-dimethylaminopropyl)carbodiimide hydrochloride); glutaraldehyde, formaldehyde, paraformaldehyde, succinaldehyde, glyoxal, methylene glycol, or any combination thereof. In some embodiments, glutaraldehyde acetals, 1,4-pyran, 2-alkoxy-3,4-dihydro-2H-pyrans (e.g., 2-ethoxy-3,4-dihydro-2H-pyran), or any combination thereof, are employed in the methods provided herein.

Non-limiting examples of crosslinking agents include homobifunctional crosslinking agents, heterobifunctional crosslinking agents, trifunctional crosslinking agents, multifunctional crosslinking agents, and combinations thereof. A homobifunctional crosslinking agent has a spacer arm with same reactive groups at both the ends. A heterobifunctional crosslinking agent has a spacer arm with different reactive groups at the two ends. A trifunctional crosslinking agent has three short spacers arms linked to a central atom, such as nitrogen, and each spacer arm ending in a reactive group. The crosslinking agents disclosed herein may crosslink amino-amino groups, amino-sulfhydryl groups, sulfhydryl-sulfhydryl groups, amino-carboxyl groups, and the like. Any crosslinking agent known in the art that crosslink proteins may be used. In addition, the crosslinking agents may be a chemical crosslinking agent or a UV-inducible crosslinking agent.

The fixing agents can be membrane permeable (e.g., membrane permeable crosslinking agents) The cleavable and/or membrane permeable crosslinking agent can comprise dithiobis(succinimidyl propionate) (DSP, Lomant's Reagent), disuccinimidyl tartrate (DST), Bis [2-(Succinimidooxycarbonyloxy)ethylj Sulfone (BSOCOES), ethylene glycol bis(succinimidyl succinate) (EGS), dimethyl 3,3'-dithiobispropionimidate (DTBP, Wang and Richard's Reagent), succinimidyl 3-(2-pyridyldithio)propionate (SPDP), succinimidyl 6-(3(2-pyridyldithio)propionamido)hexanoate (LC-SPDP), 4-succinimidyloxycarbonyl-alpha-methyl-o(2-pyridyldithio)toluene (SMPT), 3-(2-pyridyldithio)propionyl hydrazide =~ (PDPH), succinimidyl 2-((4,4'-azipentanamido)ethyl)-1,3'-dithiopropionate (SDAD, NHS-SS-Diazirine), or any combination thereof.

The method described herein can comprise reversing the fixation of the sample, which can comprise contacting the sample with an unfixing agent. The unfixing agent can be membrane permeable. The unfixing agent can comprise a thiol, hydoxylamine, periodate, a base, or any combination thereof. The unfixing agent can comprise DTT. Reversing the fixation of the sample can comprise UV photocleaving, chemical treatment, heating, enzyme treatment, or any combination thereof. Reversing the fixation of the sample can comprise lysing the sample. Lysing the sample can comprise heating, contacting the sample with a detergent, changing the pH, or any combination thereof.

Without being bound by any particular theory, it is believed that it can be advantageous to use heat for reversing formaldehyde crosslinking, and use proteinase K to dissociate (e.g., break up) protein-nucleic acid complexes that are formed during cell fixation, which can improve RNA preservation and recovery. The methods and compositions described herein can result in unexpected and superior results in fixing/permeabilizing cells for protein detection and preserve mRNA and to reverse crosslink mRNAs for sample analysis involving the detection and analysis of both proteins and RNA (e.g., RNAseq).

*Permeabilizing agents*

Disclosed herein include permeabilizing agents capable of permeabilizing the cell membrane of the sample. The permeabilizing agent can be capable of making a cell membrane permeable to the protein target-binding reagents (e.g., intracellular target-binding reagents). The permeabilizing agent can comprise a solvent, a detergent, or a surfactant, or any combination thereof. The permeabilizing agent can comprise BD Cytoperm. The permeabilizing agent can comprise a saponin or a derivative thereof, digitonin or a derivative thereof, or any combination thereof.

As used herein, "permeabilizing" a cell can refer to a treatment that reduces the integrity of a cell membrane, thereby allowing molecules, e.g., modifying agents, enzymes, antibodies, other proteins, access to the intracellular space. Permeabilization can comprise disrupting, dissolving, modifying, and/or forming pores in the lipid membrane. In some embodiments, permeabilization does not involve disruption of the cellular morphology or lysis of the cell. Permeabilization can be performed using any one or more of a variety of solvents, surfactants and/or commercially-available reagents. In some embodiments, the cells are permeabilized using an organic solvent. Examples of organic solvents include, but are not limited to, benzene, n-butanol, n-propanol, isopropanol, toluene, ether, phenylethyl alcohol, chloroform, hexane, ethanol, and acetone. In some embodiments, a surfactant, detergent or emulsifying agent is used to permeabilize a cell membrane. Non-limiting examples of permeabilizing agents include saponin, NP-40, Tween-20, triton X-100, brij 35, Duponal, digitonin, thionins, chlorpromazine, imipramine, plyethyleneimine, sodium dodecyl sulfate, sodium deoxycholate, and sodium N-lauryl-sarcosylate. In further embodiments, commercially available permeabilization reagents and kits including but not limited to Leucoperm™, PerFix-EXPOSE, PerFix-nc, Fix&Perm® kit, Cytofix/Cytoperm™ solution, and Image-iT® Fixation Permeabilization Kit. Other suitable permeabilization reagents and methods may be used and are known in the art.

Microarrays

Disclosed herein include compositions. In some embodiments, the composition comprises: an oligonucleotide array comprising a plurality of spatial regions, wherein the oligonucleotide array comprises at least 100 spots, wherein each spot of the oligonucleotide array comprises a distinct spatial region of the plurality of spatial regions, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

The composition can comprise: a cartridge, wherein the cartridge comprises at least one of: an inlet port, an outlet port, a pump, a valve, a vent, a reservoir, a sample collection chamber, a temperature control apparatus, or any combination thereof. The composition can comprise: a buffer. The composition can comprise: one or more reagents for a reverse transcription reaction, one or more reagents for an amplification reaction, or both. In some embodiments, the cartridge comprises a transparent window for optical imaging of the at least 100 microwells.

In some embodiments, a solid support/substrate can refer to a microarray (e.g., an oligonucleotide array). A microarray can comprise a plurality of polymers, e.g., oligomers, synthesized in situ or pre-synthesized and deposited on a substrate in an array pattern. Microarrays of oligomers manufactured by solid-phase DNA synthesis can have oligomer densities approaching 106/micron2. As used herein, the support-bound oligomers can be referred to as called "probes" or "oligonucleotide barcodes", which function to bind or hybridize with a sample of DNA or RNA material under test. However, the terms can be used interchangeably wherein the surface-bound oligonucleotides as targets and the solution sample of nucleic acids as probes. Further, some investigators bind the target sample under test to the microarray substrate and put the oligomer probes in solution for hybridization. Either of the "target" or "probes" can be the one that is to be evaluated by the other (thus, either one could be an unknown mixture of polynucleotides to be evaluated by binding with the other). All of these iterations are within the scope of this discussion herein. For the purpose of simplicity only, herein the probe is the surface-bound oligonucleotide of known sequence and the target is the moiety in a mobile phase (typically fluid), to be detected by the surface-bound probes. The plurality of probes and/or targets in each location (e.g., spot) in the array can be referred to as a "nucleic acid feature" or "feature." A feature is defined as a locus onto which a large number of probes and/or targets all having a similar or identical nucleotide sequence (e.g., a feature-specific spatial label) are immobilized.

An "array" can refer to an intentionally created collection of molecules which can be prepared either synthetically or biosynthetically. The molecules in the array can be identical or different from each other. The array can assume a variety of formats, e.g., libraries of soluble molecules; libraries of compounds tethered to resin beads, silica chips, or other solid supports. Array Plate or a Plate a body having a plurality of arrays in which each array can be separated from the other arrays by a physical barrier resistant to the passage of liquids and forming an area or space, referred to as a well.

The density of the microarrays can be higher than 500, 5000, 50000, or 500,000 different probes per cm$^2$. The feature size of the probes can be smaller than 500, 150, 25, 9, or 1 $\mu$m$^2$. The locations of the probes can be determined or decipherable. For example, in some arrays, the specific locations of the probes are known before binding assays. In some other arrays, the specific locations of the probes are unknown until after the assays. The probes can be immobilized on a substrate, optionally, via a linker, beads, etc.

The array can comprise features made up of oligo(dT) probes. The array can comprise features made up of gene-specific probes. In some embodiments, the array is a microarray. In some embodiments, the array is planar. In some embodiments, the array has topographical features.

Microwell Arrays

A substrate can refer to a type of solid support. A substrate can refer to a solid support that can comprise stochastic barcodes of the disclosure. A substrate can comprise a plurality of microwells. A microwell can comprise a small reaction chamber of defined volume. Disclosed herein include compositions. In some embodiments, the composition comprises: a micro-well array comprising a plurality of spatial regions, wherein the micro-well array comprises at least 100 micro-wells, wherein each micro-well has a volume ranging from about 10 $\mu$m$^3$ to about 786,000 $\mu$m$^3$, wherein each micro-well comprises a distinct spatial region, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

The microwells of the array can be fabricated in a variety of shapes and sizes. Appropriate well geometries can include, but are not limited to, cylindrical, conical, hemispherical, rectangular, or polyhedral (e.g., three dimensional geometries comprised of several planar faces, for example, hexagonal columns, octagonal columns, inverted triangular pyramids, inverted square pyramids, inverted pentagonal pyramids, inverted hexagonal pyramids, or inverted truncated pyramids). The microwells can comprise a shape that combines two or more of these geometries. For example, a microwell can be partly cylindrical, with the remainder having the shape of an inverted cone. A microwell can include two side-by-side cylinders, one of larger diameter than the other, that are connected by a vertical channel (that is, parallel to the cylinder axes) that extends the full length (depth) of the cylinders. The opening of the microwell can be at the upper surface of the substrate. The opening of the microwell can be at the lower surface of the substrate. The closed end (or bottom) of the microwell can be flat. The closed end (or bottom) of the microwell can have a curved surface (e.g., convex or concave).

Microwell dimensions can be characterized in terms of the diameter and depth of the well. As used herein, the diameter of the microwell refers to the largest circle that can be inscribed within the planar cross-section of the microwell geometry.

The diameter of the microwells can be specified in terms of absolute dimensions. The diameter of the microwells can range from about 5 to about 50 micrometers. The microwell diameter can be at least 5 micrometers, at least 10 micrometers, at least 15 micrometers, at least 20 micrometers, at least 25 micrometers, at least 30 micrometers, at least 35 micrometers, at least 40 micrometers, at least 45 micrometers, or at least 50 micrometers. The microwell diameter can be at most 50 micrometers, at most 45 micrometers, at most 40 micrometers, at most 35 micrometers, at most 30 micrometers, at most 25 micrometers, at most 20 micrometers, at most 15 micrometers, at most 10 micrometers, or at most 5 micrometers. The microwell diameter can be about 30 micrometers.

In some embodiments, the diameter of each microwell can be, or can be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, nanometer. In some embodiments, the diameter of each microwell can be, or can be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, micrometer. In some embodiments, the diameter of each microwell can be, or can be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, minimeter.

The depth of the microwells can be specified in terms of absolute dimensions. The depth of the microwells can range from about 10 to about 60 micrometers. The microwell depth can be at least 10 micrometers, at least 20 micrometers, at least 25 micrometers, at least 30 micrometers, at least 35 micrometers, at least 40 micrometers, at least 50 micrometers, or at least 60 micrometers. The microwell depth can be at most 60 micrometers, at most 50 micrometers, at most 40 micrometers, at most 35 micrometers, at most 30 micrometers, at most 25 micrometers, at most 20 micrometers, or at most 10 micrometers. The microwell depth can be about 30 micrometers.

In some embodiments, the depth of each microwell can be, or can be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, nanometers. In some embodiments, the depth of each microwell can be, or can be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, micrometers. In some embodiments, the depth of each microwell can be, or can be about, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, minimeters.

The volume of the microwells used in the methods, devices, and systems of the present disclosure can vary, for example range from about 200 micrometers$^3$ to about 120,000 micrometers$^3$. The microwell volume can be at least 200 micrometers$^3$, at least 500 micrometers$^3$, at least 1,000 micrometers$^3$, at least 10,000 micrometers$^3$, at least 25,000 micrometers$^3$, at least 50,000 micrometers$^3$, at least 100,000 micrometers$^3$, or at least 120,000 micrometers$^3$. The microwell volume can be at most 120,000 micrometers$^3$, at most 100,000 micrometers $^3$, at most 50,000 micrometers$^3$, at most 25,000 micrometers$^3$, at most 10,000 micrometers$^3$, at most 1,000 micrometers$^3$, at most 500 micrometers$^3$, or at most 200 micrometers$^3$. The microwell volume can be about 25,000 micrometers$^3$. The microwell volume can fall within any range bounded by any of these values (e.g. from about 18,000 micrometers$^3$ to about 30,000 micrometers$^3$).

In some embodiments, each of the microwells can have a volume of 10, 20, 30, 40, 50, 60, 70, 80, 90, 100, 200, 300, 400, 500, 600, 700, 800, 900, 1000, or a number between any two of these values, nanoliters. In some embodiments, each of the microwells can have a volume of 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, microliters. In some embodiments, each of the microwells can have a volume of 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, miniliters.

The wells of the microwell array can be arranged in a one dimensional, two dimensional, or three-dimensional array. A three dimensional array can be achieved, for example, by stacking a series of two or more two dimensional arrays (that is, by stacking two or more substrates comprising microwell arrays).

The pattern and spacing between microwells can vary. The microwells can be distributed according to a variety of random or non-random patterns. For example, they can be distributed entirely randomly across the surface of the array substrate, or they can be arranged in a square grid, rectangular grid, hexagonal grid, or the like. The center-to-center distance (or spacing) between wells can vary from about 15 micrometers to about 75 micrometers. In other embodiments, the spacing between wells is at least 15 micrometers, at least 20 micrometers, at least 25 micrometers, at least 30 micrometers, at least 35 micrometers, at least 40 micrometers, at least 45 micrometers, at least 50 micrometers, at least 55 micrometers, at least 60 micrometers, at least 65 micrometers, at least 70 micrometers, or at least 75 micrometers. The microwell spacing can be at most 75 micrometers, at most 70 micrometers, at most 65 micrometers, at most 60 micrometers, at most 55 micrometers, at most 50 micrometers, at most 45 micrometers, at most 40 micrometers, at most 35 micrometers, at most 30 micrometers, at most 25 micrometers, at most 20 micrometers, or at most 15 micrometers. The microwell spacing can be about 55 micrometers. The microwell spacing can fall within any range bounded by any of these values (e.g. from about 18 micrometers to about 72 micrometers).

In some embodiments, microwells can be separated from each other by no more than 0.01, 0.1, 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, micrometers. In some embodiments, the microwells can be separated from one another by no more than 1, 2, 3, 4, 5, 6, 7, 8, 9, 10, 20, 30, 40, 50, 60, 70, 80, 90, 100, 200, 300, 400, 500, 600, 700, 800, 900, 1000, or a number between any two of these values, minimeters.

In some embodiments, the microwell array can comprise 100, 200, 300, 400, 500, 600, 700, 800, 900, 1000, 2000, 3000, 4000, 5000, 6000, 7000, 8000, 9000, 10000, or a number between any two of these values, wells per inch$^2$. In some embodiments, the microwell array can comprise 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number between any two of these values, wells per cm$^2$.

The microwell array can comprise surface features including, but not limited to, domed, ridged, or peaked surface features that encircle the wells or straddle the surface between wells.

The total number of wells in the microwell array can be determined by the pattern and spacing of the wells and the overall dimensions of the array. The number of microwells in the array can vary, for example, range from about 96 to about 5,000,000 or more. The number of microwells in the array can be at least 96, at least 384, at least 1,536, at least 5,000, at least 10,000, at least 25,000, at least 50,000, at least 75,000, at least 100,000, at least 500,000, at least 1,000,000, or at least 5,000,000. The number of microwells in the array can be at most 5,000,000, at most 1,000,000, at most 75,000, at most 50,000, at most 25,000, at most 10,000, at most 5,000, at most 1,536, at most 384, or at most 96 wells. The number of microwells in the array can be about 96. The number of microwells can be about 150,000. The number of microwells in the array can fall within any range bounded by any of these values (e.g. from about 100 to 325,000).

Microwell arrays can be fabricated using any of a number of fabrication techniques. Examples of fabrication methods that can be used include, but are not limited to, bulk micromachining techniques such as photolithography and wet chemical etching, plasma etching, or deep reactive ion etching; micro-molding and micro-embossing; laser micromachining; 3D printing or other direct write fabrication processes using curable materials; and similar techniques.

Microwell arrays can be fabricated from any of a number of substrate materials. The choice of material can depend on the choice of fabrication technique, and vice versa. Examples of suitable materials can include, but are not limited to, silicon, fused-silica, glass, polymers (e.g. agarose, gelatin, hydrogels, polydimethylsiloxane (PDMS; elastomer), polymethylmethacrylate (PMMA), polycarbonate (PC), polypropylene (PP), polyethylene (PE), high density polyethylene (HDPE), polyimide, cyclic olefin polymers (COP), cyclic olefin copolymers (COL), polyethylene terephthalate (PET), epoxy resins, thiol-ene based resins, metals or metal films (e.g. aluminum, stainless steel, copper, nickel, chromium, and titanium), and the like. A hydrophilic material can be desirable for fabrication of the microwell arrays (e.g. to enhance wettability and minimize non-specific binding of cells and other biological material). Hydrophobic materials that can be treated or coated (e.g. by oxygen plasma treatment, or grafting of a polyethylene oxide surface layer) can also be used. The use of porous, hydrophilic materials for the fabrication of the microwell array can be desirable in order to facilitate capillary wicking/venting of entrapped air bubbles in the device. The microwell array can be fabricated from a single material. The microwell array can comprise two or more different materials that have been bonded together or mechanically joined.

Microwell arrays can be fabricated using substrates of any of a variety of sizes and shapes. For example, the shape (or footprint) of the substrate within which microwells are fabricated can be square, rectangular, circular, or irregular in shape. The footprint of the microwell array substrate can be similar to that of a microtiter plate. The footprint of the microwell array substrate can be similar to that of standard microscope slides, e.g. about 75 mm long x 25 mm wide (about 3" long x 1" wide), or about 75 mm long x 50 mm wide (about 3" long x 2" wide). The thickness of the substrate within which the microwells are fabricated can range from about 0.1 mm thick to about 10 mm thick, or more. The thickness of the microwell array substrate can be at least 0.1 mm thick, at least 0.5 mm thick, at least 1 mm thick, at least 2 mm thick, at least 3 mm thick, at least 4 mm thick, at least 5 mm thick, at least 6 mm thick, at least 7 mm thick, at least 8 mm thick, at least 9 mm thick, or at least 10 mm thick. The thickness of the microwell array substrate can be at most 10 mm thick, at most 9 mm thick, at most 8 mm thick, at most 7 mm thick, at most 6 mm thick, at most 5 mm thick, at most 4 mm thick, at most 3 mm thick, at most 2 mm thick, at most 1 mm thick, at most 0.5 mm thick, or at most 0.1 mm thick. The thickness of the microwell array substrate can be about 1 mm thick. The thickness of the microwell array substrate can be any value within these ranges, for example, the thickness of the microwell array substrate can be between about 0.2 mm and about 9.5 mm.

A variety of surface treatments and surface modification techniques can be used to alter the properties of microwell array surfaces. Examples can include, but are not limited to, oxygen plasma treatments to render hydrophobic material surfaces more hydrophilic, the use of wet or dry etching techniques to smooth (or roughen) glass and silicon surfaces, adsorption or grafting of polyethylene oxide or other polymer layers (such as pluronic), or bovine serum albumin to substrate surfaces to render them more hydrophilic and less prone to non-specific adsorption of biomolecules and cells, the use of silane reactions to graft chemically-reactive functional groups to otherwise inert silicon and glass surfaces, etc. Photodeprotection techniques can be used to selectively activate chemically-reactive functional groups at specific locations in the array structure, for example, the selective addition or activation of chemically-reactive functional groups such as primary amines or carboxyl groups on the inner walls of the microwells can be used to covalently couple oligonucleotide probes, peptides, proteins, or other biomolecules to the walls of the microwells. The choice of surface treatment or surface modification utilized can depend both or either on the type of surface property that is desired and on the type of material from which the microwell array is made.

Flow Cells and Cartridges

Substrates provided herein (e.g., microwell array substrate) can be packaged within a flow cell that provides for convenient interfacing with the rest of the fluid handling system and facilitates the exchange of fluids, e.g. cell and solid support suspensions, lysis buffers, rinse buffers, etc., that are delivered to the microwell array and/or emulsion droplet. Design features can include: (i) one or more inlet ports for introducing cell samples, solid support suspensions, or other assay reagents, (ii) one or more microwell array chambers designed to provide for uniform filling and efficient fluid-exchange while minimizing back eddies or dead zones, and (iii) one or more outlet ports for delivery of fluids to a sample collection point or a waste reservoir. The design of the flow cell can further include features for creating uniform flow velocity profiles, i.e. "plug flow". In some embodiments, the flow cell can enclose or incorporate more than one microwell array substrate. In some embodiments, the integrated microwell array / flow cell assembly can constitute a fixed component of the system. In some embodiments, the microwell array/flow cell assembly can be removable from the instrument.

In some embodiments of the system, the substrate (e.g., microwell array), with or without an attached flow cell, can be packaged within a consumable cartridge that interfaces with an instrument system. Design features of cartridges can include (i) one or more inlet ports for creating fluid connections with the instrument or manually introducing cell samples, bead suspensions, or other assay reagents into the cartridge, (ii) one or more bypass channels, i.e. for self-metering of cell samples and bead suspensions, to avoid overfilling or back flow, (iii) one or more integrated microwell array / flow cell assemblies, or one or more chambers within which the microarray substrate(s) are positioned, (iv) integrated miniature pumps or other fluid actuation mechanisms for controlling fluid flow through the device, (v) integrated miniature valves (or other containment mechanisms) for compartmentalizing preloaded reagents (for example, bead suspensions) or controlling fluid flow through the device, (vi) one or more vents for providing an escape path for trapped air, (vii) one or more sample and reagent waste reservoirs, (viii) one or more outlet ports for creating fluid connections with the instrument or providing a processed sample collection point, (ix) mechanical interface features for reproducibly positioning the removable, consumable cartridge with respect to the instrument system, and for providing access so that external magnets can be brought into close proximity with the microwell array, (x) integrated temperature control components or a thermal interface for providing good thermal contact with the instrument system, and (xi) optical interface features, e.g. a transparent window, for use in optical interrogation of the microwell array.

Blocking Oligonucleotides and Decov Oligonucleotides

The disclosed methods and compositions can reduce the amplification and/or extension of undesirable nucleic acid species in the sample, and/or allow selective removal of undesirable nucleic acid species in the sample, using, for example, the methods and compositions described in US20200190564 entitled "SELECTIVE EXTENSION IN SINGLE CELL WHOLE TRANSCRIPTOME ANALYSIS", the content of which is incorporated herein by reference in its entirety.

In some embodiments, the methods disclosed herein comprise providing a blocking oligonucleotide that specifically binds to at least one of the one or more undesirable nucleic acid species. The blocking oligonucleotides can be provided at any point during the methods disclosed herein so that they can reduce the amplification and/or extension of the undesirable nucleic acid species. For example, the blocking oligonucleotides can be provided before, during or after the extension step, before or during the amplification step, before, during or after contacting the plurality of oligonucleotide barcodes with the plurality of nucleic acid target molecules for hybridization, or any combination thereof. A "blocking oligonucleotide" as used herein refers to a nucleic acid molecule that can specifically bind to at least one of the one or more undesirable nucleic acid species, whereby the specifically binding between the blocking oligonucleotide and the one or more undesirable nucleic acid species can reduce the amplification or extension (e.g., reverse transcription) of the one or more undesirable nucleic acid species. For example, the blocking oligonucleotide can comprise a nucleic acid sequence capable of hybridizing with one or more undesirable nucleic acid species. In some embodiments, a plurality of blocking oligonucleotides can be provided. The plurality of blocking oligonucleotides can specifically bind to at least 1, at least 2, at least 5, at least 10, at least 50, at least 100, at least 200, at least 500, at least 1000, or more, or a range between any two of these values, of the one or more undesirable nucleic acid species. The location at which a blocking oligonucleotide specifically binds to an undesirable nucleic acid species can vary. For example, blocking oligonucleotide can specifically binds to a sequence close to the 5' end of the undesirable nucleic acid species. In some embodiments, the blocking oligonucleotide can specifically bind to within 1 nt, 3 nt, 5 nt, 8 nt, 10 nt, 15 nt, 20 nt, 25 nt, 30 nt, 35 nt, 40 nt, 50 nt, 75 nt, 100 nt, 200 nt, 300 nt, 400 nt, 500 nt, or 1000 nt, or a range between any two of these values, of the 5' end of at least one of the one or more undesirable nucleic acid species. In some embodiments, blocking oligonucleotide can specifically binds to a sequence close to the 3' end of the undesirable nucleic acid species. For example, the blocking oligonucleotide can specifically bind to within 1 nt, 3 nt, 5 nt, 10 nt, 15 nt, 20 nt, 25 nt, 30 nt, 35 nt, 40 nt, 45 nt, 50 nt, 75 nt, 100 nt, 200 nt, 300 nt, 400 nt, 500 nt, or 1000 nt, or more, or a range between of any two of these values, of the 3' end of at least one of the one or more undesirable nucleic acid species. In some embodiments, blocking oligonucleotide can specifically binds to a sequence in the middle portion of the undesirable nucleic acid species. For example, the blocking oligonucleotide can specifically bind to within 1 nt, 3 nt, 5 nt, 10 nt, 20 nt, 25 nt, 30 nt, 35 nt, 40 nt, 45 nt, 50 nt, 75 nt, 100 nt, 200 nt, 300 nt, 400 nt, 500 nt, or 1000 nt, or more, or a range between any two of these values, from the middle point of at least one of the one or more undesirable nucleic acid species. In some embodiments, the undesirable nucleic acid species is an mRNA species. The blocking oligonucleotide can bind, or not bind, to the poly(A) tail of the mRNA species. In some embodiments, the blocking oligonucleotide specifically bind to the region adjacent to the poly(A) tail of the mRNA species. In some embodiments, the blocking oligonucleotide specifically bind to the poly(A) tail and the region adjacent to the poly(A) tail of the mRNA species. In some embodiments, the one or more undesirable nucleic acid species are mRNA molecules and the blocking oligonucleotide specific binds to within 1 nt, 3 nt, 5 nt, 8 nt, 10 nt, or a range between any two of these values, of the 3' poly(A) tail of the one or more undesirable nucleic acid species.

The blocking oligonucleotide can comprise various sequence components. For example, the blocking oligonucleotide can comprise (i) a sequence that specifically binds to the at least one of the one or more undesirable nucleic acid species and/or (ii) the sequence, or a subsequence, of the target binding region.

In some embodiments, the specifically binding between the blocking oligonucleotide and the undesirable nucleic acid species can reduce the amplification and/or extension of the undesirable nucleic acid species by at least 10%, at least 20%, at least 30%, at least 40%, at least 50%, at least 60%, at least 70%, at least 80%, at least 90%, at least 95%, at least 98%, at least 99%, or more.

It is contemplated that the blocking oligonucleotide may reduce the amplification and/or extension of the undesirable nucleic acid species by, for example, forming a hybridization complex with the undesirable nucleic acid species having a high melting temperature ($T_m$), by not being able to function as a primer for a reverse transcriptase, a polymerase, or a combination thereof. In some embodiments, the blocking oligonucleotide can have a $T_m$ that is, is about, is at least, 40°C, 50°C, 55°C, 60°C, 65°C, 70°C, 75°C, 80°C, 85°C, 90°C, 95°C, or a range between any two of these values. In some embodiments, the blocking oligonucleotide can reduce the amplification and/or extension of the undesirable nucleic acid species by competing with the amplification and/or extension primers for hybridization with the undesirable nucleic acid species. In some embodiments, the blocking oligonucleotide is unable to function as a primer for a reverse transcriptase, or a polymerase, or both.

The blocking oligonucleotide can comprise one or more non-natural nucleotides. Non-natural nucleotides can be, for example, photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). In some embodiments, the blocking oligonucleotide is a chimeric oligonucleotide, such as an LNA/PNA/DNA chimera, an LNA/DNA chimera, a PNA/DNA chimera, a GNA/DNA chimera, a TNA/DNA chimera, or any combination thereof.

The length of the blocking oligonucleotide can vary. For example, the melting temperature ($T_m$) of a blocking oligonucleotide can be modified, in some embodiments, by adjusting the length of the blocking oligonucleotide. For example, a blocking oligonucleotide can have a length that is, is about, is less than, is more than, 4 nt, 6 nt, 8 nt, 10 nt, 12 nt, 15 nt, 20 nt, 21 nt, 25 nt, 30 nt, 35 nt, 40 nt, 45 nt, 50 nt, 60 nt, 70 nt, 80 nt, 90 nt, 100 nt, 150 nt, 200 nt, or a range between any two of the above values. In some embodiments, the blocking oligonucleotide is, or is about, 8 nt to 100 nt long. In some embodiments, the blocking oligonucleotide is, or is about, 10 nt to 50 nt long. In some embodiments, the blocking oligonucleotide is, or is about, 12 nt to 21 nt long. In some embodiments, the blocking oligonucleotide is, or is about, 20 nt to 30 nt long, for example 25 nt long.

In some embodiments, the $T_m$ of a blocking oligonucleotide is modified by the number of DNA residues in the blocking oligonucleotide that comprises an LNA/DNA chimera or a PNA/DNA chimera. For example, a blocking oligonucleotide that comprises an LNA/DNA chimera or a PNA/DNA chimera can have a percentage of DNA residues that is, is about, is less than, is more than, 10%, 15%, 20%, 25%, 30%, 35%, 40%, 45%, 50%, 60 %, 70%, 80%, 90%, or a range between any two of the above values.

In some embodiments, a blocking oligonucleotide can be designed to be incapable of functioning as a primer or probe for an amplification and/or extension reaction. For example, the blocking oligonucleotide may be incapable of function as a primer for a reverse transcriptase or a polymerase. For example, a blocking oligonucleotide that comprises an LNA/DNA chimera or a PNA/DNA chimera can be designed to have a certain percentage of LNA or PNA residues, or to have LNA or PNA residues on certain locations, such as close to or at the 3' end, 5' end, or in the middle portion of the oligonucleotide. In some embodiments, a blocking oligonucleotide that comprises an LNA/DNA chimera or a PNA/DNA chimera can have a percentage of LNA or PNA residues that is, is about, is less than, is more than, 10%, 15%, 20%, 25%, 30%, 35%, 40%, 45%, 50%, 60%, 70%, 80%, 90%, or a range between any two of the above values.

In some embodiments, a blocking oligonucleotide can specifically bind to at least one or more undesirable nucleic acid species and can incapable of functioning as a primer or probe for an amplification and/or extension reaction due to a lack of hybridization of its 3' end to the one or more undesirable nucleic acid species. In some embodiments, a blocking oligonucleotide can comprise a 3' non-annealing region. The 3' non-annealing region can be located at the extreme 3' end of the blocking oligonucleotide. The 3' non-annealing region can comprise one or more nucleotides which are non-complementary to a undesirable nucleic acid species specifically bound by the 5' region of the blocking oligonucleotide. The blocking oligonucleotide can be incapable of being extendable by a reverse transcriptase or a DNA polymerase due to the absence of hybridization of the 3' non-annealing region to the undesirable nucleic acid species.

In some embodiments, the blocking oligonucleotide comprises a 5' region that can specifically bind to at least one or more undesirable nucleic acid species and a 3' non-annealing region. In some such embodiments, the 5' region specifically binds to the 3' end of the at least one or more undesirable nucleic acid species. In some embodiments, the blocking oligonucleotide comprises a 5' poly(dT) region that binds an undesirable nucleic acid species (e.g., a housekeeping mRNA), a 3' non-annealing region that does not bind the undesirable nucleic acid species, and a intervening region that specifically binds the undesirable nucleic acid species. In some embodiments, the length of the 3' non-annealing region relative to the total length of the blocking oligonucleotide is, is about, is less than, is more than, 10%, 15%, 20%, 25%, 30%, 35%, 40%, 45%, 50%, 60%, 70%, 80%, 90%, or a range between any two of the above values. The sequence of the 3' non-annealing region can be shared across different blocking oligonucleotides targeting different undesirable nucleic acid species. In some such embodiments, the 3' non-annealing region comprises a sequence that is configured to not hybridize to some or all undesirable nucleic acid species. In other embodiments, the sequence of the 3' non-annealing region is configured to prevent hybridization to a particular undesirable nucleic acid species.

The GC content of the 3' non-annealing region of the blocking oligonucleotide can vary depending on the embodiment. In some embodiments, a higher GC content prevents non-specific binding of the 3' non-annealing region to an undesirable nucleic acid species. In some embodiments, the GC content of the 3' non-annealing region is, is about, is less than, is more than, 10%, 15%, 20%, 25%, 30%, 35%, 40%, 45%, 50%, 60%, 70%, 80%, 90%, or a range between any two of the above values. The 3' non-annealing region can comprise secondary structure (e.g., a hairpin) that can prevent extension of the blocking oligonucleotide. In some embodiments, the 3' non-annealing region is configured to prevent intramolecular hybridization, intermolecular hybridization with other blocking oligonucleotides, and/or secondary structure formation. The 3' non-annealing region can comprise only natural nucleotides. Alternatively, the 3' non-annealing region can comprise one or more non-natural nucleotides.

The length of the 3' non-annealing region can vary. For example, a 3' non-annealing region can have a length that is, is about, is less than, is more than, 4 nt, 6 nt, 8 nt, 10 nt, 12 nt, 15 nt, 20 nt, 21 nt, 25 nt, 30 nt, 35 nt, 40 nt, 45 nt, 50 nt, 60 nt, 70 nt, 80 nt, 90 nt, 100 nt, 150 nt, 200 nt, or a range between any two of the above values. In some embodiments, the 3' non-annealing region is, or is about, 1 nt to 100 nt long. In some embodiments, the 3' non-annealing region is, or is about, 1 nt to 50 nt long. In some embodiments, the 3' non-annealing region is, or is about, 1 nt to 21 nt long. In some embodiments, the 3' non-annealing region is, or is about, 1 nt to 10 nt long. In some embodiments, the 3' non-annealing region is, or is about, 5 nt long.

The degree of non-complementarity between the 3' non-annealing region of the blocking oligonucleotide and the undesirable nucleic acid species can vary. In some embodiments, the 3' non-annealing region has perfect non-complementarity with the sequence of the undesirable nucleic acid species adjacent to the sequence specifically bound by the blocking oligonucleotide. In some embodiments, the non-complementarity between the 3' non-annealing region and the region of the undesirable nucleic acid species 5' adjacent to the sequence specifically bound by the blocking oligonucleotide is, is about, is less than, is more than, 10%, 15%, 20%, 25%, 30%, 35%, 40%, 45%, 50%, 60%, 70%, 80%, 91%, 92%, 93%, 94%, 95%, 96%, 97%, 98%, 99%, 100%, or a range between any two of the above values.

In some embodiments, the methods disclosed herein can comprise removing the hybridized complex formed between the blocking oligonucleotide and the undesirable nucleic acid species. For example, the blocking oligonucleotides can comprise an affinity moiety. The affinity moiety can be a functional group selected from the group consisting of biotin, streptavidin, heparin, an aptamer, a click-chemistry moiety, digoxigenin, primary amine(s), carboxyl(s), hydroxyl(s), aldehyde(s), ketone(s), and any combination thereof. In some embodiments, the affinity moiety is biotin. In some embodiments, the blocking oligonucleotide can be immobilized to a solid support having a binding partner for the affinity moiety through the affinity moiety. In some embodiments, the binding partner is streptavidin.

A blocking oligonucleotide can bind, for example specifically bind, to one or more undesirable nucleic acid species. In some embodiments, the blocking oligonucleotide can bind to two or more undesirable nucleic acid species. The method disclosed herein, in some embodiments, comprises providing blocking oligonucleotide(s) that specifically bind to two or more undesirable nucleic acid species in the sample. For example, the method can comprise blocking oligonucleotide(s) that specifically bind to at least 5, 10, 15, 20, 30, 50, 60, 70, 80, 90, 100, 150, 200, 250, 300, 350, 400, 500, or more, or a range between any two of these values, undesirable nucleic acid species in the sample.

The methods and compositions provided herein can be employed in concert with blocking reagents, such as those described in U.S. Patent Application Number 63/239,367, filed on August 31, 2021, entitled "USE OF DECOY POLYNUCLEOTIDES IN SINGLE CELL MULTIOMICS", the content of which is incorporated herein by reference in its entirety. The blocking reagent can comprise, for example, (a) one or more oligonucleotides capable of hybridizing to at least a portion of the protein target-binding reagent specific oligonucleotides (e.g., AbSeq oligonucleotides, cellular component binding reagent specific oligonucleotides); (b) one or more decoy oligonucleotides capable of hybridizing to at least one of the one or more non-target nucleic acid, or any combination thereof. The sample can be fixed and/or permeabilized in the presence of the blocking reagent, the sample can contact the blocking reagent after the sample is fixed and/or permeabilized, or both. In some embodiments, the method described herein comprises contacting the cells with the blocking reagent (e.g., decoy oligonucleotides) after the sample is contacted with the fixative and/or the permeabilizing agent. The methods and compositions provided herein can be employed in concert with the methods and compositions described in U.S. Patent Application Number 63/239,369, filed on August 31, 2021, entitled "RNA PRESERVATION AND RECOVERY FROM FIXED CELLS", the content of which is incorporated herein by reference in its entirety. In some embodiments, the decoy oligonucleotides (e.g., those shown in Table 1) do not comprise a 3' modification. In some embodiments, the 3' modification is capable of reducing or preventing polymerase extension of the decoy oligonucleotide (e.g., a 3' dideoxy-C modification (ddC)). In some embodiments, the decoy oligonucleotide is configured to prevent its 3' extension via a polymerase by comprising, for example, an extension blocker. The 3' modification can be one or more extension blockers. The 3' modification can be a sequence configured to not anneal to the target-binding region of an oligonucleotide barcode. The extension blocker can comprise one or more 2'-O-methyl (2'OM) RNA nucleotides. The extension blocker can comprise one or more of an abasic site, a stable abasic site, a chemically trapped abasic site, or any combination thereof. In some embodiments, the stable abasic site comprises 1',2'-dideoxy. In some embodiments, the chemically trapped abasic site comprises an abasic site reacted with alkoxy amine or sodium borohydride. In some embodiments, the abasic site comprises an apurinic site, an apyrimidinic site, or both. In some embodiments, the abasic site is generated by an alkylating agent or an oxidizing agent. In some embodiments, the one or extension blockers comprise: one or more nitroindoles, one or more inosines, one or more acridines, one or more 2-aminopurines, one or more 2-6-diaminopurines, one or more 5-bromo-deoxyuridines, one or more inverted thymidines (inverted dTs), one or more inverted dideoxy-thymidines (ddTs), one or more dideoxy-cytidines (ddCs), one or more 5-m ethyl cytidines, one or more 5-hydroxymethylcytidines, one or more 2'-O-Methyl RNA bases, one or more unmethylated RNA bases, one or more Iso- deoxycytidines (Iso-dCs), one or more Iso-deoxyguanosines (Iso-dGs), one or more C3 ($OC_3H_6OPO_3$) groups, one or more photocleavable (PC) [$OC_3H_6$-C(o)NHCH$_2$-C$_6$H$_3$NO$_2$-CH(CH$_3$)OPO$_3$] groups, one or more hexandiol groups, one or more spacer 9 (iSp9) [$(OCH_2CH_2)_3OPO_3$] groups, one or more spacer 18 (iSpl8) [$(OCH_2CH_{26}OPO_3$] groups, or any combination thereof. In some embodiments the decoy oligonucleotide is configured to simulate intracellular target-binding reagent specific oligonucleotides and/or cell surface target-binding reagent specific oligonucleotides and thereby bind undesirable nucleic acid species that would otherwise bind to the intracellular target-binding reagent specific oligonucleotides and/or cell surface target-binding reagent specific oligonucleotides in the disclosed methods. In some embodiments, the decoy oligonucleotides provided herein comprise, do not comprise, and/or comprise modified versions of, one or more elements of intracellular target-binding reagent specific oligonucleotides and/or cell surface target-binding reagent specific oligonucleotides provided herein, such as, for example, an alignment sequence, a UMI, an antibody-specific barcode sequence, a primer adapter, a universal PCR handle, and/or a sequence configured to bind a target-binding region of an oligonucleotide barcode (e.g., a poly(A) tail). The systems, methods, compositions, and kits provided herein can, in some embodiments, be employed in concert with the systems, methods, compositions, and kits described in PCT Application Publication No. WO/2021/163374, the content of which is incorporated herein by reference in its entirety.

Without being bound by any particular theory, it is believed that the decoy oligonucleotide can, for example, prevent or reduce undesirable non-specific binding of the protein target-binding reagent specific oligonucleotide to non-target cellular/endogenous nucleic acid, therefore to reduce noises in the methods. For example, the decoy oligonucleotide can be capable of hybridizing to at least one of the one or more non-target nucleic acid, or a portion thereof. A decoy oligonucleotide can comprise one or more common features with a protein target-binding reagent specific oligonucleotides. For example, the decoy oligonucleotide can comprise the same or substantial same AbSeq barcode as the protein target-binding reagent specific oligonucleotides. In some embodiments, the AbSeq barcode is about 20 to 50 nucleotides, for example 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of these values, nucleotides. In some embodiments, the decoy oligonucleotide does not have AbSeq barcode. In some embodiments, the decoy oligonucleotide comprises a sequence identical to or substantially similar to a sequence of the protein target-binding reagent specific oligonucleotides. For example, the decoy oligonucleotide can have a sequence having, having about, having at least about, 100%, 99%, 98%, 97%, 97%, 96%, 95%, 94%, 93%, 92%, 91%, 90%, 85%, 80%, 75%, 70%, 65%, 60%, or a number or a range between any two of these values, sequence identity to the protein target-binding reagent specific oligonucleotides. Such a sequence of the decoy oligonucleotide can be, can be about, can be at least, or can be at most, 3, 5, 8, 10, 12, 14, 15, 18, 20, 25, 30, 35, 40, or a number or a range between any two of these values, nucleotides in length. In some embodiments, the decoy oligonucleotide has, has about, or has at most, 5%, 10%, 15%, 20%, 25%, 30%, 35%, 40%, 45%, 50%, a number or a range between any two of these values, sequence identity to the protein target-binding reagent specific oligonucleotides.

In some embodiments, the decoy oligonucleotide comprises a random sequence region in which there is at least one G or C in every four, five, six, or seven nucleotide stretch (i.e., four, five, six, or seven consecutive nucleotides). In some embodiments, the decoy oligonucleotide does not comprise any poly(dT) or poly(dA) regions with more than three, four, five or six consecutive Ts or As. In some embodiments, the decoy oligonucleotide comprises an AbSeq barcode sequence and a random sequence of about 12-15 nucleotides in length.

The decoy oligonucleotide can comprise one or more modified nucleotides, a 5' modification, a 3' modification, or any combination thereof. The 3' modification can be, for example 3' dideoxy-C modification (ddC). The 5' modification can be, for example, 5' Amino Modifier C12 modification (5AmMC12). The length of the decoy oligonucleotide can vary, for example the decoy oligonucleotide can be, or be about, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, or a range between any two of these values, nucleotides in length. The decoy oligonucleotide can comprise one or more UMI or UMI regions. In some embodiments, the decoy oligonucleotide does not comprise UMI. In some embodiments, the decoy oligonucleotide comprises one or more random sequence regions (e.g.,. one, two, three, four random sequence regions). In some embodiments, the decoy oligonucleotide does not comprise a random sequence. The length of the UMI can vary, for example, the UMI can be, or be about, 3, 4, 5, 6, 7, 8, 9, 10, 11, 12, 13, 14, 15, 16, 17, 18, 19, 20, or a range between any two of these values, nucleotides in length. The length of the random sequence region can vary, for example, the random sequence region can be, be about, be at most, or be at least, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or a range between any two of these values, nucleotides in length. In some embodiments, the decoy oligonucleotide comprises a random sequence region of, or of about, 30 nucleotides in length, and wherein the random sequence region has at least one G or C in every 6-nucleotide stretch.

In some embodiments, the blocking reagent comprise one or more oligonucleotide having a sequence complementary to a portion of the sequence of a protein target-binding reagent specific oligonucleotide. In some embodiments, the method described herein comprising contacting the plurality of protein target-binding reagent specific oligonucleotide with the cells in the presence of the blocking reagent. In some embodiments, the decoy oligonucleotide comprises a random sequence having substantially the same length of the molecular label of one or more of the protein target-binding reagent specific oligonucleotides.

Methods, compositions and kits described herein can reduce noises caused by non-specific binding of barcode oligonucleotides (e.g., the antibody-specific oligonucleotide in the oligonucleotide-conjugated antibodies) to non-target cellular proteins (e.g., cell surface proteins and intracellular proteins). It can be advantageous to use the methods, compositions and kits disclosed herein in sample analysis involving when a lot of cellular/endogenous nucleic acids are exposed. As described herein, noises caused by non-specific binding can be reduced (e.g., partial reduction, near complete reduction, and complete reduction) by the use of decoy oligonucleotides.

In some embodiments, the sample is contacted with one, two or three unique decoy oligonucleotides and incubated for a desirable period of time (e.g., about 1, 2, 3, 4, 5, 6, 7, 8, 9, 10, 12, 15, 20, 30, 60, or a number or a range between any two of these values, minutes). For example, the decoy oligonucleotide can result in, result in about, or results in at least, 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, or a number or a range between any two of these values, reduction in non-specific binding to non-target nucleic acid. In some embodiments, the decoy oligonucleotide can result in, result in about, or results in at least, 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100%, or a number or a range between any two of these values, reduction in noises in a proteogenomics or other proteomic workflow using AbOs.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

One skilled in the art will appreciate that, for this and other processes and methods disclosed herein, the functions performed in the processes and methods can be implemented in differing order. Furthermore, the outlined steps and operations are only provided as examples, and some of the steps and operations can be optional, combined into fewer steps and operations, or expanded into additional steps and operations without detracting from the essence of the disclosed embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

The invention also provides the following numbered embodiments:

1. A method for determining the spatial location and copy number of a nucleic acid target in a sample, comprising:

providing a substrate comprising a plurality of spatial regions, wherein a plurality of oligonucleotide barcodes are associated with each of the spatial regions, wherein each of the oligonucleotide barcodes comprises a universal sequence, a molecular label, a target-binding region capable of hybridizing to a nucleic acid target, and a predetermined spatial label, wherein oligonucleotide barcodes of the same spatial region comprise the same spatial label, and wherein oligonucleotide barcodes of the different spatial regions comprise different spatial labels;
contacting the substrate with a sample comprising copies of a nucleic acid target, wherein the contacting comprises contacting the plurality of the spatial regions with the sample such that each distinct spatial region contacts a distinct spatial location of the sample;
extending the plurality of oligonucleotide barcodes hybridized to the copies of a nucleic acid target to generate a plurality of barcoded nucleic acid molecules each comprising a sequence complementary to at least a portion of the nucleic acid target;
obtaining sequencing data comprising a plurality of sequencing reads of the plurality of barcoded nucleic acid molecules, or products thereof, wherein each of the plurality of sequencing reads comprises a spatial label sequence, a molecular label sequence, and a subsequence of the nucleic acid target; and

for each unique spatial label sequence, which is associated a distinct spatial region of the substrate and thereby a distinct spatial location of the sample, counting the number of molecular labels with distinct sequences associated with a nucleic acid target to determine the copy number of the nucleic acid target at each spatial location of the sample.

2. The method of embodiment 1, wherein the sample comprises a plurality of cellular component targets, further comprising:

contacting a plurality of cellular component-binding reagents with the sample, wherein each of the plurality of cellular component-binding reagents comprises a cellular component-binding reagent specific oligonucleotide comprising a unique identifier sequence for the cellular component-binding reagent, and wherein the cellular component-binding reagent is capable of specifically binding to at least one of the plurality of cellular component targets;

extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence;

obtaining sequencing data comprising a plurality of sequencing reads of the plurality of barcoded cellular component-binding reagent specific oligonucleotides, or products thereof, wherein each of the plurality of sequencing reads comprises a spatial label sequence, a molecular label sequence, and at least a portion of the unique identifier sequence; and

for each unique spatial label sequence, which is associated a distinct spatial region of the substrate and thereby a distinct spatial location of the sample, counting the number of molecular labels with distinct sequences associated with a unique identifier sequence to determine the number of copies of at least one cellular component target of the plurality of cellular component targets at each spatial location of the sample.

3. The method of any one of embodiments 1-2, wherein at least a portion of the contacting step is performed in the presence in the presence of extension reagents, optionally the entire contacting step is performed in the presence in the presence of the extension reagents, further optionally the contacting and extension steps are simultaneous, optionally the sample and substrate are in contact during the extension step, optionally the extension is performed in situ.

4. The method of any one of embodiments 1-3, wherein the extension reagents comprise reverse transcription reagents, optionally reverse transcription reagents comprise a reverse transcriptase and dNTPs.

5. The method of any one of embodiments 1-4, wherein the method comprises a denaturing step, optionally denaturing separates the nucleic acid target from the barcoded nucleic acid molecule and/or the cellular component-binding reagent specific oligonucleotide from the barcoded cellular component-binding reagent specific oligonucleotide.

6. The method of any one of embodiments 1-5, wherein the contacting step comprises an overlay of the substrate on the sample.

7. The method of any one of embodiments 1-6, comprising separating the substrate from the sample from the substrate after the contacting step.

8. The method of any one of embodiments 1-7, wherein the method (i) comprises fixing the sample prior to the contacting step or (ii) does not comprise fixing the sample prior to the contacting step.

9. The method of any one of embodiments 1-8, wherein the sample is physically divided or is intact during the contacting step.

10. The method of any one of embodiments 1-9, wherein the spatial locations of the nucleic acid targets in the sample are on a surface of the sample, inside the sample, subcellularly in the sample, or any combination thereof.

11. The method of any one of embodiments 1-10, wherein the sample comprises a plurality of cells, optionally the nucleic acid targets are associated with the plurality of cells.

12. The method of embodiment 11, wherein the plurality of cells comprise one or more cell types, optionally said one or more cell types are selected from the group consisting of: brain cells, heart cells, cancer cells, circulating tumor cells, organ cells, epithelial cells, metastatic cells, benign cells, primary cells, and circulatory cells, or any combination thereof.

13. The method of any one of embodiments 1-12, wherein the sample comprises a tissue, a cell monolayer, fixed cells, a tissue section, or any combination thereof.

14. The method of any one of embodiments 1-13, wherein the sample comprises a biological sample, a clinical sample, an environmental sample, a biological fluid, a tissue, a tissue section derived from a subject, or any combination thereof, optionally the subject is a human, a mouse, a dog, a rat, or a vertebrate.

15. The method of any one of embodiments 1-14, further comprising determining genotype, phenotype, or one or more genetic mutations of the subject based on the spatial location of the nucleic acid targets in the sample.

16. The method of any one of embodiments 1-15, further comprising predicting susceptibility of the subject to one or more diseases.

17. The method of any one of embodiments 1-16, wherein at least one of the one or more diseases is cancer or a hereditary disease.

18. The method of any one of embodiments 1-17, further comprising determining cell types of the plurality of cells in the sample.

19. The method of any one of embodiments 1-18, wherein a drug is chosen based on predicted responsiveness of the cell types of the plurality of cells in the sample.

20. The method of any one of embodiments 1-19, comprising imaging the sample, optionally imaging the sample after the contacting step, optionally the imaging generates imaging data.

21. The method of any one of embodiments 1-20, wherein imaging the sample comprises staining the sample with a stain, wherein the stain is a fluorescent stain, a negative stain, an antibody stain, or any combination thereof, optionally staining comprises Immunocytochemistry (ICC), Immunohistochemistry (IHC), Immunofluorescence (IF), or any combination thereof.

22. The method of any one of embodiments 1-21, wherein imaging comprises microscopy, confocal microscopy, time-lapse imaging microscopy, fluorescence microscopy, multi-photon microscopy, quantitative phase microscopy, surface enhanced Raman spectroscopy, videography, manual visual analysis, automated visual analysis, or any combination thereof.

23. The method of any one of embodiments 1-22, further comprising associating the imaging data and sequencing data of one or more spatial locations of the sample.

24. The method of any one of embodiments 1-23, comprising correlation analysis of the imaging data and the sequencing data of the spatial locations, optionally the correlation analysis identifies one or more of the following: candidate biomarkers, candidate therapeutic agents, candidate doses of therapeutic agents, and/or cellular targets of candidate therapeutic agents.

25. The method of any one of embodiments 1-24, wherein said imaging produces an image that is used to construct a map of a physical representation of said sample, optionally said map is two dimensional or three dimensional.

26. The method of any one of embodiments 1-25, comprising mapping the nucleic acid targets and/or cellular component targets onto the map of the sample.

27. The method of any one of embodiments 1-26, the method comprising isolating one or more barcoded nucleic acid molecules corresponding to a spatial location of interest, optionally the isolating comprises selective hybridization and pull-down of one or more barcoded nucleic acid molecules comprising the unique spatial label of a spatial region corresponding to a spatial location of interest.

28. The method of any one of embodiments 1-27, wherein the oligonucleotide barcode comprises a target-binding region comprising a capture sequence, optionally the target-binding region comprises a poly(dT) region.

29. The method of any one of embodiments 1-28, wherein the cellular component-binding reagent specific oligonucleotide comprises a sequence complementary to the capture sequence configured to capture the cellular component-binding reagent specific oligonucleotide, optionally the sequence complementary to the capture sequence comprises a poly(dA) region.

30. The method of any one of embodiments 1-29, wherein the nucleic acid target comprises a nucleic acid molecule.

31. The method of any one of embodiments 1-30, wherein the nucleic acid molecule comprises ribonucleic acid (RNA), messenger RNA (mRNA), microRNA, small interfering RNA (siRNA), RNA degradation product, RNA comprising a poly(A) tail, a sample indexing oligonucleotide, a cellular component-binding reagent specific oligonucleotide, or any combination thereof.

32. The method of any one of embodiments 1-31, wherein each of the spatial regions comprises a plurality of oligonucleotide barcodes configured to hybridize to a panel of nucleic acid targets.

33. The method of any one of embodiments 1-32, wherein the cellular component-binding reagent specific oligonucleotide comprises a second universal sequence.

34. The method of any one of embodiments 1-33, wherein obtaining sequence information of the plurality of barcoded cellular component-binding reagent specific oligonucleotides, or products thereof, comprises:

amplifying the plurality of barcoded cellular component-binding reagent specific oligonucleotides, or products thereof, using a primer capable of hybridizing to the first universal sequence, or a complement thereof, and a primer capable of hybridizing to the second universal sequence, or a complement thereof, to generate a plurality of amplified barcoded cellular component-binding reagent specific oligonucleotides; and

obtaining sequencing data of the plurality of amplified barcoded cellular component-binding reagent specific oligonucleotides, or products thereof.

35. The method of any one of embodiments 1-34, wherein obtaining sequencing data comprises attaching sequencing adaptors to (i) the plurality of barcoded nucleic acid molecules, or products thereor, and/or (ii) the plurality of barcoded cellular component-binding reagent specific oligonucleotides, or products thereof.

36. The method of any one of embodiments 1-35, comprising after contacting the plurality of cellular component-binding reagents with the sample, removing one or more cellular component-binding reagents of the plurality of cellular component-binding reagents that are not contacted with the sample, optionally removing the one or more cellular component-binding reagents not contacted with the sample comprises: removing the one or more cellular component-binding reagents not contacted with the respective at least one of the plurality of cellular component targets.

37. The method of any one of embodiments 1-36, wherein the cellular component target comprises an intracellular protein, a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof.

38. The method of any one of embodiments 1-37, wherein extending the plurality of oligonucleotide barcodes comprises extending the plurality of oligonucleotide barcodes using a reverse transcriptase and/or a DNA polymerase lacking at least one of 5' to 3' exonuclease activity and 3' to 5' exonuclease activity, optionally the DNA polymerase comprises a Klenow Fragment, further optionally the reverse transcriptase comprises a viral reverse transcriptase, optionally wherein the viral reverse transcriptase is a murine leukemia virus (MLV) reverse transcriptase or a Moloney murine leukemia virus (MMLV) reverse transcriptase.

39. The method of any one of embodiments 1-38, wherein less than about 5 percent of nucleic acid targets are mapped to an incorrect spatial location due to diffusion of said nucleic acid target from a starting spatial location.

40. The method of any one of embodiments 1-39,

wherein each of the plurality of oligonucleotide barcodes comprises a substrate linker functional group,
wherein each of the plurality of substrates comprises a substrate functional group, and
wherein the substrate functional group and the substrate linker functional group are associated with each other.

41. The method of embodiment 40, wherein the substrate linker functional group and the substrate functional group are individually selected from the group consisting of C6, biotin, streptavidin, primary amine(s), aldehyde(s), ketone(s), and any combination thereof.

42. The method of any one of embodiments 1-41, wherein the oligonucleotide barcode is associated with the substrate through a substrate linker.

43. The method of embodiment 42, wherein the substrate linker comprises a carbon chain, optionally the carbon chain comprises 2-30 carbons, and further optionally the carbon chain comprises 12 carbons.

44. The method of any one of embodiments 42-43, wherein the substrate linker comprises 5' amino modifier C12 (5AmMC12), or a derivative thereof.

45. The method of any one of embodiments 1-44, wherein the spatial label is 6-60 nucleotides in length.

46. The method of any one of embodiments 1-45, wherein the oligonucleotide barcode:

is 50-500 nucleotides in length;
is attached to the substrate;
is covalently attached to the substrate;
is conjugated to the substrate;
is conjugated to the substrate through a chemical group selected from the group consisting of a UV photocleavable group, a streptavidin, a biotin, an amine, and a combination thereof,
is non-covalently attached to the substrate; and/or
is configured to be detachable from the substrate.

47. The method of any one of embodiments 1-46, comprising dissociating the oligonucleotide barcode from the substrate, and optionally dissociating the oligonucleotide barcode from the substrate comprises detaching the oligonucleotide barcode from the substrate by UV photocleaving, chemical treatment, heating, enzyme treatment, or any combination thereof.

48. The method of embodiment 47, wherein the dissociating occurs after barcoding the oligonucleotide barcodes.

49. The method of any one of embodiments 1-48, wherein the oligonucleotide barcode is configured to be non-detachable from the substrate.

50. The method of any one of embodiments 1-49, wherein the oligonucleotide barcode is conjugated to the substrate by a 1,3-dipolar cycloaddition reaction, a hetero-Diels-Alder reaction, a nucleophilic substitution reaction, a non-aldol type carbonyl reaction, an addition to carbon-carbon multiple bond, an oxidation reaction, a click reaction, or any combination thereof.

51. The method of any one of embodiments 1-50, wherein at least one oligonucleotide barcode of the plurality of oligonucleotide barcodes is immobilized on the substrate, partially immobilized on the substrate, enclosed in the substrate, partially enclosed in the substrate, or a combination thereof.

52. The method of any one of embodiments 1-51, wherein the substrate comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof.

53. The method of any one of embodiments 1-52, wherein the target-binding region comprises a gene-specific sequence, an oligo(dT) sequence, a random multimer, or any combination thereof.

54. The method of any one of embodiments 1-53, wherein each spatial label of the plurality of oligonucleotide barcodes comprise at least 6 nucleotides.

55. The method of any one of embodiments 1-54, wherein each molecular label of the plurality of oligonucleotide barcodes comprises at least 6 nucleotides.

56. The method of any one of embodiments 1-55, wherein the substrate comprises a solid support.

57. The method of any one of embodiments 1-56, wherein the solid support comprises a planar surface.

58. The method of any one of embodiments 1-57, wherein the substrate comprises an oligonucleotide array comprising a plurality of spots, and wherein each spot of the oligonucleotide array comprises a distinct spatial region of the plurality of spatial regions.

59. The method of any one of embodiments 1-58, wherein the substrate comprises a microwell array, and wherein each microwell of the microwell array comprises a distinct spatial region of the plurality of spatial regions.

60. The method of any one of embodiments 1-59, comprising, prior to contacting a plurality of cellular component-binding reagents with the sample and/or contacting the substrate with the sample, contacting the sample with a blocking reagent, one or more decoy oligonucleotides, and/or one or more blocking oligonucleotides.

61. The method of any one of embodiments 1-60, wherein contacting a plurality of cellular component-binding reagents with the sample is conducted in the presence of a blocking reagent.

62. The method of any one of embodiments 1-61, wherein the blocking reagent comprises a plurality of oligonucleotides complementary to at least a portion of the cellular component-binding reagent specific oligonucleotides.

63. The method of any one of embodiments 1-62, wherein the blocking reagent comprises an antibody or a fragment thereof derived from a first species, and wherein the blocking reagent comprises sera derived from the first species.

64. The method of any one of embodiments 1-63, wherein the sample comprises one or more non-target nucleic acids, wherein the blocking reagent comprises a plurality of decoy oligonucleotides capable of hybridizing to at least one of the one or more non-target nucleic acids.

65. The method of any one of embodiments 1-64, wherein each of the plurality of decoy oligonucleotides are capable of hybridizing to at least a portion of a non-target nucleic acid.

66. The method of any one of embodiments 1-65, wherein the decoy oligonucleotide comprises a sequence complementary to at least a portion of a non-target nucleic acid.

67. The method of any one of embodiments 1-66, wherein the decoy oligonucleotide comprises a sequence identical to or substantially similar to a sequence of the cellular component-binding reagents specific oligonucleotides.

68. The method of embodiment 67, wherein the sequence is 3-40 nucleotides in length.

69. The method of any one of embodiments 1-68, wherein the decoy oligonucleotide has at most 50% sequence identity to the cellular component-binding reagent specific oligonucleotides.

70. The method of any one of embodiments 1-69, wherein the decoy oligonucleotide does not comprise a UMI.

71. The method of any one of embodiments 1-69, wherein the decoy oligonucleotide comprises a random sequence, and optionally the random sequence is about four, five, six, seven, eight, nine, ten, eleven, twelve, thirteen, fourteen, or fifteen nucleotides in length.

72. The method of any one of embodiments 1-71, wherein the decoy oligonucleotide does not comprise any sequence having more than four, five, six, or seven consecutive Ts or As.

73. The method of any one of embodiments 1-72, wherein the decoy oligonucleotide comprise at least one G or C in every four, five, six, or seven consecutive nucleotides.

74. The method of any one of embodiments 1-73, wherein the decoy oligonucleotide comprises one or more modified nucleotides.

75. The method of any one of embodiments 1-74, wherein the decoy oligonucleotide comprises a 5' modification, and optionally the 5' modification comprises a 5' Amino Modifier C12 modification (5AmMC12).

76. The method of any one of embodiments 1-75, wherein the decoy oligonucleotide comprises a 3' modification, and optionally the 3' modification comprises a 3' dideoxy-C modification (ddC).

77. The method of any one of embodiments 1-76, wherein the decoy oligonucleotide is 30 to 65 nucleotides in length.

78. The method of any one of embodiments 1-77, wherein the sample comprises one or more undesirable nucleic acid species, the method comprising:

contacting a blocking oligonucleotide with the sample, wherein the blocking oligonucleotide specifically binds to at least one of the one or more undesirable nucleic acid species; whereby the extension of the at least one of the one or more undesirable nucleic acid species is reduced by the blocking oligonucleotide

79. The method of embodiment 78, wherein the blocking oligonucleotide:

is contacted with the sample before the substrate is contacted with the sample; is contacted with the sample after the substrate is contacted with the sample; and/or is contacted with the sample when the substrate is contacted with the sample.

80. The method of any one of embodiments 78-79, wherein the blocking oligonucleotide comprises: (i) a sequence that specifically binds to the at least one of the one or more undesirable nucleic acid species, and (ii) the sequence, or a subsequence, of the target binding region.

81. The method of any one of embodiments 78-80, wherein the blocking oligonucleotide comprises (i) a sequence that specifically binds to the at least one of the one or more undesirable nucleic acid species, (ii) the sequence, or a subsequence, of the target binding region, and (iii) a sequence that does not hybridize to the at least one of the one or more undesirable nucleic acid species.

82. The method of any one of embodiments 78-81, wherein the blocking oligonucleotide comprises a 3' non-annealing region configured to not anneal to the one or more undesirable nucleic acid species; and optionally the non-complementarity between the 3' non-annealing region and the region of the undesirable nucleic acid species 5' adjacent to the sequence specifically bound by the blocking oligonucleotide is at least 50%, is at least 60%, is at least 70%, is at least 80%, is at least 90%, is at least 95%, or is about 100%.

83. The method of embodiment 82, wherein the 3' non-annealing region is 1 nt to 100 nt long, is 1 nt to 50 nt long, is 1 nt to 21 nt long, is 1 nt to 10 nt long, or is about 5 nt long.

84. The method of any one of embodiments 78-83, wherein the blocking oligonucleotide is a locked nucleic acid (LNA), a peptide nucleic acid (PNA), a DNA, an LNA/PNA chimera, an LNA/DNA chimera, or a PNA/DNA chimera.

85. The method of any one of embodiments 78-83, wherein the blocking oligonucleotide does not comprise non-natural nucleotides.

86. The method of any one of embodiments 78-85, comprising providing blocking oligonucleotides that specifically bind to two or more undesirable nucleic acid species, optionally at least 10 or to at least 100 undesirable nucleic acid species, in the sample.

87. The method of any one of embodiments 78-86, wherein the blocking oligonucleotide:

has a $T_m$ of at least 50 °C, of at least 60 °C, or of at least 70 °C; is unable to function as a primer for a reverse transcriptase or a polymerase; and/or is 8 nt to 100 nt long, is 10 nt to 50 nt long, is 12 nt to 21 nt long, is 20 nt to 30 nt long, or is about 25 nt long.

88. The method of any one of embodiments 78-87, wherein the one or more undesirable nucleic acid species amounts to about 50%, to about 60%, to about 70%, or to about 80% of the nucleic acid content of the sample.

89. The method of any one of embodiments 78-88, wherein the undesirable nucleic acid species is selected from the group consisting of ribosomal RNA, mitochondrial RNA, genomic DNA, intronic sequence, high abundance sequence, and a combination thereof.

90. The method of any one of embodiments 78-89, wherein the blocking oligonucleotides specifically binds to within 100 nt, to within 50 nt, or to within 25 nt of the 3' end of the one or more undesirable nucleic acid species.

91. The method of any one of embodiments 78-90, wherein the blocking oligonucleotide specifically binds to within 100 nt of the 5' end of the one or more undesirable nucleic acid species, or the blocking oligonucleotide specifically binds to within 100 nt of the middle of the one or more undesirable nucleic acid species.

92. The method of any one of embodiments 78-91, wherein the one or more undesirable nucleic acid species are mRNA molecules and the blocking oligonucleotide specific binds to within 10 nt of the 3' poly(A) tail of the one or more undesirable nucleic acid species.

93. A composition comprising:

a micro-well array comprising a plurality of spatial regions, wherein the micro-well array comprises at least 100 micro-wells, wherein each micro-well has a volume ranging from about 10 $\mu m^3$ to about

786,000 $\mu$m³, wherein each micro-well comprises a distinct spatial region, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

94. A composition comprising:

an oligonucleotide array comprising a plurality of spatial regions,
wherein the oligonucleotide array comprises at least 100 spots, wherein each spot of the oligonucleotide array comprises a distinct spatial region of the plurality of spatial regions, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

95. The composition of any one of embodiments 93-94, further comprising a cartridge, wherein the cartridge comprises at least one of: an inlet port, an outlet port, a pump, a valve, a vent, a reservoir, a sample collection chamber, a temperature control apparatus, or any combination thereof.

96. The composition of any one of embodiments 93-95, comprising a buffer.

97. The composition of any one of embodiments 93-96, comprising one or more reagents for a reverse transcription reaction, one or more reagents for an amplification reaction, or both.

98. The composition of any one of embodiments 93-97, wherein the cartridge comprises a transparent window for optical imaging of the at least 100 microwells.

**Claims**

1. A method for determining the spatial location and copy number of a nucleic acid target in a sample, comprising:

providing a substrate comprising a plurality of spatial regions, wherein a plurality of oligonucleotide barcodes are associated with each of the spatial regions, wherein each of the oligonucleotide barcodes comprises a universal sequence, a molecular label, a target-binding region capable of hybridizing to a nucleic acid target, and a predetermined spatial label, wherein oligonucleotide barcodes of the same spatial region comprise the same spatial label, and wherein oligonucleotide barcodes of the different spatial regions comprise different spatial labels;
contacting the substrate with a sample comprising copies of a nucleic acid target and a plurality of cellular component targets, wherein the contacting comprises contacting the plurality of the spatial regions with the sample such that each distinct spatial region contacts a distinct spatial location of the sample;
contacting a plurality of cellular component-binding reagents with the sample, wherein each of the plurality of cellular component-binding reagents comprises a cellular component-binding reagent specific oligonucleotide comprising a unique identifier sequence for the cellular component-binding reagent, and wherein the cellular component-binding reagent is capable of specifically binding to at least one of the plurality of cellular component targets;
extending the plurality of oligonucleotide barcodes hybridized to the copies of a nucleic acid target to generate a plurality of barcoded nucleic acid molecules each comprising a sequence complementary to at least a portion of the nucleic acid target;
extending the plurality of oligonucleotide barcodes hybridized to the cellular component-binding reagent specific oligonucleotides to generate a plurality of barcoded cellular component-binding reagent specific oligonucleotides each comprising a sequence complementary to at least a portion of the unique identifier sequence;
obtaining sequencing data comprising a plurality of sequencing reads of the plurality of barcoded nucleic acid molecules, or products thereof, wherein each of the plurality of sequencing reads comprises a spatial label sequence, a molecular label sequence, and a subsequence of the nucleic acid target;
obtaining sequencing data comprising a plurality of sequencing reads of the plurality of barcoded cellular component-binding reagent specific oligonucleotides, or products thereof, wherein each of the plurality of sequencing reads comprises a spatial label sequence, a molecular label sequence, and at least a portion of the unique identifier sequence;
for each unique spatial label sequence, which is associated a distinct spatial region of the substrate and thereby a distinct spatial location of the sample, counting the number of molecular labels with distinct sequences associated with a nucleic acid target to determine the copy number of the nucleic acid target at each spatial location of the sample.

for each unique spatial label sequence, which is associated a distinct spatial region of the substrate and thereby a distinct spatial location of the sample, counting the number of molecular labels with distinct sequences associated with a unique identifier sequence to determine the number of copies of at least one cellular component target of the plurality of cellular component targets at each spatial location of the sample.

2. The method of claim 1, wherein at least a portion of the contacting step is performed in the presence in the presence of extension reagents, optionally the entire contacting step is performed in the presence in the presence of the extension reagents, further optionally the contacting and extension steps are simultaneous, optionally the sample and substrate are in contact during the extension step, optionally the extension is performed in situ.

3. The method of any one of claims 1-2, wherein:

(a) the extension reagents comprise reverse transcription reagents, optionally reverse transcription reagents comprise a reverse transcriptase and dNTPs;
(b) the method comprises a denaturing step, optionally denaturing separates the nucleic acid target from the barcoded nucleic acid molecule and/or the cellular component-binding reagent specific oligonucleotide from the barcoded cellular component-binding reagent specific oligonucleotide;
(c) the contacting step comprises an overlay of the substrate on the sample;
(d) the method comprises separating the substrate from the sample from the substrate after the contacting step; and/or
(e) the method:

(i) comprises fixing the sample prior to the contacting step; or
(ii) does not comprise fixing the sample prior to the contacting step; and/or

(f) the sample is physically divided or is intact during the contacting step; and/or
(g) the spatial locations of the nucleic acid targets in the sample are on a surface of the sample, inside the sample, subcellularly in the sample, or any combination thereof.

4. The method of any one of claims 1-3, wherein the sample comprises:

(a) a plurality of cells, optionally wherein:

(i) the nucleic acid targets are associated with the plurality of cells; and/or
(ii) the plurality of cells comprise one or more cell types, optionally said one or more cell types are selected from the group consisting of: brain cells, heart cells, cancer cells, circulating tumor cells, organ cells, epithelial cells, metastatic cells, benign cells, primary cells, and circulatory cells, or any combination thereof, and/or

(b) a tissue, a cell monolayer, fixed cells, a tissue section, or any combination thereof; and/or
(c) a biological sample, a clinical sample, an environmental sample, a biological fluid, a tissue, a tissue section derived from a subject, or any combination thereof, optionally the subject is a human, a mouse, a dog, a rat, or a vertebrate.

5. The method of any one of claims 1-4, wherein:

(a) the method further comprises determining genotype, phenotype, or one or more genetic mutations of the subject based on the spatial location of the nucleic acid targets in the sample;
(b) the method further comprises predicting susceptibility of the subject to one or more diseases, optionally wherein at least one of the one or more diseases is cancer or a hereditary disease;
(c) the method further comprises determining cell types of the plurality of cells in the sample; and/or
(d) a drug is chosen based on predicted responsiveness of the cell types of the plurality of cells in the sample.

6. The method of any one of claims 1-5, comprising imaging the sample, optionally wherein:

(a) imaging the sample after the contacting step, optionally the imaging generates imaging data, optionally wherein:

(i) the method further comprises associating the imaging data and sequencing data of one or more spatial locations of the sample; and/or
(ii) the method comprises correlation analysis of the imaging data and the sequencing data of the spatial locations, optionally the correlation analysis identifies one or more of the following: candidate biomarkers, candidate therapeutic agents, candidate doses of therapeutic agents, and/or cellular targets of candidate therapeutic agents; and/or

(b) imaging the sample comprises staining the sample with a stain, wherein the stain is a fluorescent stain, a negative stain, an antibody stain, or any combination thereof, optionally staining comprises Immunocytochemistry (ICC), Immunohistochemistry (IHC), Immunofluorescence (IF), or any combination thereof,

(c) imaging comprises microscopy, confocal microscopy, time-lapse imaging microscopy, fluorescence microscopy, multi-photon microscopy, quantitative phase microscopy, surface enhanced Raman spectroscopy, videography, manual visual analysis, automated visual analysis, or any combination thereof; and/or

(d) said imaging produces an image that is used to construct a map of a physical representation of said sample, optionally:

    (i) said map is two dimensional or three dimensional; and/or
    (ii) comprising mapping the nucleic acid targets and/or cellular component targets onto the map of the sample.

7. The method of any one of claims 1-6, wherein:

    (a) the method comprises isolating one or more barcoded nucleic acid molecules corresponding to a spatial location of interest, optionally the isolating comprises selective hybridization and pull-down of one or more barcoded nucleic acid molecules comprising the unique spatial label of a spatial region corresponding to a spatial location of interest;
    (b) the oligonucleotide barcode comprises a target-binding region comprising a capture sequence, optionally the target-binding region comprises a poly(dT) region;
    (c) the cellular component-binding reagent specific oligonucleotide comprises a sequence complementary to the capture sequence configured to capture the cellular component-binding reagent specific oligonucleotide, optionally the sequence complementary to the capture sequence comprises a poly(dA) region;
    (d) the nucleic acid target comprises a nucleic acid molecule, optionally wherein the nucleic acid molecule comprises ribonucleic acid (RNA), messenger RNA (mRNA), microRNA, small interfering RNA (siRNA), RNA degradation product, RNA comprising a poly(A) tail, a sample indexing oligonucleotide, a cellular component-binding reagent specific oligonucleotide, or any combination thereof;
(e) each of the spatial regions comprises a plurality of oligonucleotide barcodes configured to hybridize to a panel of nucleic acid targets;
(f) the cellular component-binding reagent specific oligonucleotide comprises a second universal sequence;
(g) obtaining sequence information of the plurality of barcoded cellular component-binding reagent specific oligonucleotides, or products thereof, comprises:

    amplifying the plurality of barcoded cellular component-binding reagent specific oligonucleotides, or products thereof, using a primer capable of hybridizing to the first universal sequence, or a complement thereof, and a primer capable of hybridizing to the second universal sequence, or a complement thereof, to generate a plurality of amplified barcoded cellular component-binding reagent specific oligonucleotides; and
    obtaining sequencing data of the plurality of amplified barcoded cellular component-binding reagent specific oligonucleotides, or products thereof; and/or

(h) obtaining sequencing data comprises attaching sequencing adaptors to (i) the plurality of barcoded nucleic acid molecules, or products thereor, and/or (ii) the plurality of barcoded cellular component-binding reagent specific oligonucleotides, or products thereof; and/or
(i) the method comprises after contacting the plurality of cellular component-binding reagents with the sample, removing one or more cellular component-binding reagents of the plurality of cellular component-binding reagents that are not contacted with the sample, optionally removing the one or more cellular component-binding reagents not contacted with the sample comprises: removing the one or more cellular component-binding reagents not contacted with the respective at least one of the plurality of cellular component targets; and/or
(j) the cellular component target comprises an intracellular protein, a carbohydrate, a lipid, a protein, an extracellular protein, a cell-surface protein, a cell marker, a B-cell receptor, a T-cell receptor, a major histocompatibility complex, a tumor antigen, a receptor, an intracellular protein, or any combination thereof; and/or
(k) extending the plurality of oligonucleotide barcodes comprises extending the plurality of oligonucleotide barcodes using a reverse transcriptase and/or a DNA polymerase lacking at least one of 5' to 3' exonuclease activity and 3' to 5' exonuclease activity, optionally the DNA polymerase comprises a Klenow Fragment, further optionally the reverse transcriptase comprises a viral reverse transcriptase, optionally wherein the viral reverse transcriptase is a murine leukemia virus (MLV) reverse transcriptase or a Moloney murine leukemia virus (MMLV) reverse transcriptase; and/or

(l) less than about 5 percent of nucleic acid targets are mapped to an incorrect spatial location due to diffusion of said nucleic acid target from a starting spatial location; and/or

(m) each of the plurality of oligonucleotide barcodes comprises a substrate linker functional group, wherein each of the plurality of substrates comprises a substrate functional group, and wherein the substrate functional group and the substrate linker functional group are associated with each other, optionally wherein the substrate linker functional group and the substrate functional group are individually selected from the group consisting of C6, biotin, streptavidin, primary amine(s), aldehyde(s), ketone(s), and any combination thereof; and/or

(n) the oligonucleotide barcode is associated with the substrate through a substrate linker, optionally wherein:

    (i) the substrate linker comprises a carbon chain, optionally the carbon chain comprises 2-30 carbons, and further optionally the carbon chain comprises 12 carbons; and/or

    (ii) the substrate linker comprises 5' amino modifier C12 (5AmMC12), or a derivative thereof; and/or

    (o) the spatial label is 6-60 nucleotides in length.

8. The method of any one of claims 1-7, wherein the oligonucleotide barcode:

    is 50-500 nucleotides in length;
    is attached to the substrate;
    is covalently attached to the substrate;
    is conjugated to the substrate;
    is conjugated to the substrate through a chemical group selected from the group consisting of a UV photocleavable group, a streptavidin, a biotin, an amine, and a combination thereof;
    is non-covalently attached to the substrate; and/or
    is configured to be detachable from the substrate.

9. The method of any one of claims 1-8, wherein:

(a) the method comprises dissociating the oligonucleotide barcode from the substrate, and optionally dissociating the oligonucleotide barcode from the substrate comprises detaching the oligonucleotide barcode from the substrate by UV photocleaving, chemical treatment, heating, enzyme treatment, or any combination thereof;

(b) the dissociating occurs after barcoding the oligonucleotide barcodes;

(c) the oligonucleotide barcode is configured to be non-detachable from the substrate;

(d) the oligonucleotide barcode is conjugated to the substrate by a 1,3-dipolar cycloaddition reaction, a hetero-Diels-Alder reaction, a nucleophilic substitution reaction, a non-aldol type carbonyl reaction, an addition to carbon-carbon multiple bond, an oxidation reaction, a click reaction, or any combination thereof; and/or

(e) at least one oligonucleotide barcode of the plurality of oligonucleotide barcodes is immobilized on the substrate, partially immobilized on the substrate, enclosed in the substrate, partially enclosed in the substrate, or a combination thereof; and/or

(f) the substrate comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof; and/or

(g) the target-binding region comprises a gene-specific sequence, an oligo(dT) sequence, a random multimer, or any combination thereof; and/or

(h) each spatial label of the plurality of oligonucleotide barcodes comprise at least 6 nucleotides;

(i) each molecular label of the plurality of oligonucleotide barcodes comprises at least 6 nucleotides;

(j) the substrate comprises a solid support, optionally wherein the solid support comprises a planar surface;

(k) the substrate comprises an oligonucleotide array comprising a plurality of spots, and wherein each spot of the oligonucleotide array comprises a distinct spatial region of the plurality of spatial regions;

(l) the substrate comprises a microwell array, and wherein each microwell of the microwell array comprises a distinct spatial region of the plurality of spatial regions;

(m) the method comprises, prior to contacting a plurality of cellular component-binding reagents with the sample and/or contacting the substrate with the sample, contacting the sample with a blocking reagent, one or more decoy oligonucleotides, and/or one or more blocking oligonucleotides; and/or

(n) contacting a plurality of cellular component-binding reagents with the sample is conducted in the presence of a blocking reagent;

(o) the blocking reagent comprises a plurality of oligonucleotides complementary to at least a portion of the cellular component-binding reagent specific oligonucleotides;

(p) the blocking reagent comprises an antibody or a fragment thereof derived from a first species, and wherein the blocking reagent comprises sera derived from the first species;

(q) the sample comprises one or more non-target nucleic acids, wherein the blocking reagent comprises a plurality of decoy oligonucleotides capable of hybridizing to at least one of the one or more non-target nucleic acids; and/or

(r) each of the plurality of decoy oligonucleotides are capable of hybridizing to at least a portion of a non-target nucleic acid.

10. The method of any one of claims 1-9, wherein the decoy oligonucleotide:

(a) comprises a sequence complementary to at least a portion of a non-target nucleic acid;

(b) comprises a sequence identical to or substantially similar to a sequence of the cellular component-binding reagents specific oligonucleotides, optionally wherein the sequence is 3-40 nucleotides in length;

(c) has at most 50% sequence identity to the cellular component-binding reagent specific oligonucleotides;

(d) does not comprise a UMI;

(e) comprises a random sequence, and optionally the random sequence is about four, five, six, seven, eight, nine, ten, eleven, twelve, thirteen, fourteen, or fifteen nucleotides in length;

(f) does not comprise any sequence having more than four, five, six, or seven consecutive Ts or As;

(g) comprise at least one G or C in every four, five, six, or seven consecutive nucleotides;

(h) comprises one or more modified nucleotides;

(i) comprises a 5' modification, and optionally the 5' modification comprises a 5' Amino Modifier C12 modification (5AmMC12);

(j) comprises a 3' modification, and optionally the 3' modification comprises a 3' dideoxy-C modification (ddC); and/or

(k) is 30 to 65 nucleotides in length.

11. The method of any one of claims 1-10, wherein the sample comprises one or more undesirable nucleic acid species, the method comprising:

contacting a blocking oligonucleotide with the sample, wherein the blocking oligonucleotide specifically binds to at least one of the one or more undesirable nucleic acid species;

whereby the extension of the at least one of the one or more undesirable nucleic acid species is reduced by the blocking oligonucleotide

12. The method of claim 11, wherein:

(a) the blocking oligonucleotide:

(i) is contacted with the sample before the substrate is contacted with the sample;

(ii) is contacted with the sample after the substrate is contacted with the sample; and/or

(iii) is contacted with the sample when the substrate is contacted with the sample; and/or

(b) the blocking oligonucleotide comprises: (i) a sequence that specifically binds to the at least one of the one or more undesirable nucleic acid species, and (ii) the sequence, or a subsequence, of the target binding region; and/or

(c) the blocking oligonucleotide comprises (i) a sequence that specifically binds to the at least one of the one or more undesirable nucleic acid species, (ii) the sequence, or a subsequence, of the target binding region, and (iii) a sequence that does not hybridize to the at least one of the one or more undesirable nucleic acid species; and/or

(d) the blocking oligonucleotide comprises a 3' non-annealing region configured to not anneal to the one or more undesirable nucleic acid species; and optionally:

(i) the non-complementarity between the 3' non-annealing region and the region of the undesirable nucleic acid species 5' adjacent to the sequence specifically bound by the blocking oligonucleotide is at least 50%, is at least 60%, is at least 70%, is at least 80%, is at least 90%, is at least 95%, or is about 100%; and/or

(ii) the 3' non-annealing region is 1 nt to 100 nt long, is 1 nt to 50 nt long, is 1 nt to 21 nt long, is 1 nt to 10 nt long, or is about 5 nt long; and/or

(e) the blocking oligonucleotide is a locked nucleic acid (LNA), a peptide nucleic acid (PNA), a DNA, an LNA/PNA chimera, an LNA/DNA chimera, or a PNA/DNA chimera;

(f) the blocking oligonucleotide does not comprise non-natural nucleotides;

(g) the method comprises providing blocking oligonucleotides that specifically bind to two or more undesirable nucleic acid species, optionally at least 10 or to at least 100 undesirable nucleic acid species, in the sample; and/or
(h) the blocking oligonucleotide:

(i) has a $T_m$ of at least 50 °C, of at least 60 °C, or of at least 70 °C;
(ii) is unable to function as a primer for a reverse transcriptase or a polymerase; and/or
(iii) is 8 nt to 100 nt long, is 10 nt to 50 nt long, is 12 nt to 21 nt long, is 20 nt to 30 nt long, or is about 25 nt long; and/or

(j) the one or more undesirable nucleic acid species amounts to about 50%, to about 60%, to about 70%, or to about 80% of the nucleic acid content of the sample;
(k) the undesirable nucleic acid species is selected from the group consisting of ribosomal RNA, mitochondrial RNA, genomic DNA, intronic sequence, high abundance sequence, and a combination thereof;
(l) the blocking oligonucleotides specifically binds to within 100 nt, to within 50 nt, or to within 25 nt of the 3' end of the one or more undesirable nucleic acid species;
(m) the blocking oligonucleotide specifically binds to within 100 nt of the 5' end of the one or more undesirable nucleic acid species, or the blocking oligonucleotide specifically binds to within 100 nt of the middle of the one or more undesirable nucleic acid species; and/or
(n) the one or more undesirable nucleic acid species are mRNA molecules and the blocking oligonucleotide specific binds to within 10 nt of the 3' poly(A) tail of the one or more undesirable nucleic acid species.

13. A composition comprising:

a micro-well array comprising a plurality of spatial regions,
wherein the micro-well array comprises at least 100 micro-wells, wherein each micro-well has a volume ranging from about 10 $\mu m^3$ to about 786,000 $\mu m^3$, wherein each micro-well comprises a distinct spatial region, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

14. A composition comprising:

an oligonucleotide array comprising a plurality of spatial regions,
wherein the oligonucleotide array comprises at least 100 spots, wherein each spot of the oligonucleotide array comprises a distinct spatial region of the plurality of spatial regions, wherein a plurality of oligonucleotide barcodes are associated with each of the spatial regions, wherein each of the oligonucleotide barcodes comprises a universal sequence, a molecular label, a target-binding region, and a predetermined spatial label, wherein oligonucleotide barcodes of the same spatial region comprise the same spatial label, and wherein oligonucleotide barcodes of the different spatial regions comprise different spatial labels.

15. The composition of any one of claims 13-14, wherein:

(a) further comprising a cartridge, wherein the cartridge comprises:

(i) at least one of: an inlet port, an outlet port, a pump, a valve, a vent, a reservoir, a sample collection chamber, a temperature control apparatus, or any combination thereof; and/or
(ii) a transparent window for optical imaging of the at least 100 microwells; and/or

(b) the composition comprises a buffer; and/or
(c) the composition comprises one or more reagents for a reverse transcription reaction, one or more reagents for an amplification reaction, or both.

| bead | 5' amine | Universal label | Dimension label | Sample label | Cell label | Spatial label | Molecular label | Target-binding region |
|------|----------|-----------------|-----------------|--------------|------------|---------------|-----------------|----------------------|

108

104

**FIG. 1**

**FIG. 2**

EP 4 717 782 A2

FIG. 3

5' amine | Universal label | Spatial label | Molecular label | Target-binding region

Tissue section

400

402

400a

404

406

Tissue section

**FIG. 4A**

FIG. 4B

**FIG. 4C**

| 5' amine | Universal label | Spatial label | Molecular label | Target-binding region |
|---|---|---|---|---|

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63239897
- US 20110160078
- US 20150299784
- WO 2015031691 A
- US 20180088112 A
- US 20180346970 A
- US 20200109437 A
- US 20190338278 A
- US 091639
- US 677012
- US 20200190564 A
- US 63239367
- US 63239369
- WO 2021163374 A

**Non-patent literature cited in the description**

- **SINGLETON et al.** Dictionary of Microbiology and Molecular Biology. J. Wiley & Sons, 1994
- **SAMBROOK et al.** Molecular Cloning, A Laboratory Manual. Cold Spring Harbor Press, 1989
- **FU et al.** *Proc Natl Acad Sci U.S.A.*, 31 May 2011, vol. 108 (22), 9026-31
- **FAN et al.** *Science*, 06 February 2015, vol. 347 (6222), 1258367